(19) Europäisches Patentamt
European Patent Office
Office européen des brevets

(11) EP 4 451 061 A1

(12) EUROPEAN PATENT APPLICATION
published in accordance with Art. 153(4) EPC

(43) Date of publication:
**23.10.2024 Bulletin 2024/43**

(21) Application number: **21968294.5**

(22) Date of filing: **22.12.2021**

(51) International Patent Classification (IPC):
***G03F 7/004*** *(2006.01)* ***C07C 323/20*** *(2006.01)*
***C07C 323/21*** *(2006.01)*

(52) Cooperative Patent Classification (CPC):
**C07C 323/20; C07C 323/21; G03F 7/004**

(86) International application number:
**PCT/KR2021/019566**

(87) International publication number:
**WO 2023/113085 (22.06.2023 Gazette 2023/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.12.2021 KR 20210181068**

(71) Applicant: **Dongjin Semichem Co., Ltd.**
**Incheon 22824 (KR)**

(72) Inventors:
- **KIM, Jaehyun**
  **Hwaseong-si, Gyeonggi-do 18635 (KR)**
- **HUR, Myoung Hyun**
  **Hwaseong-si, Gyeonggi-do 18635 (KR)**
- **KIM, Jeong Sik**
  **Hwaseong-si, Gyeonggi-do 18635 (KR)**
- **YOO, Min Ja**
  **Hwaseong-si, Gyeonggi-do 18635 (KR)**
- **LEE, Hyung Kun**
  **Hwaseong-si, Gyeonggi-do 18635 (KR)**
- **JI, Chanhyuk**
  **Hwaseong-si, Gyeonggi-do 18635 (KR)**
- **JANG, Gyeonghun**
  **Hwaseong-si, Gyeonggi-do 18635 (KR)**
- **HA, Jeongmin**
  **Hwaseong-si, Gyeonggi-do 18635 (KR)**

(74) Representative: **Grünecker Patent- und Rechtsanwälte**
**PartG mbB**
**Leopoldstraße 4**
**80802 München (DE)**

# (54) NOVEL PHOTOACID GENERATOR, PHOTORESIST COMPOSITION, AND PHOTORESIST PATTERN FORMATION METHOD

(57) The present disclosure relates to: a photoacid generator comprising an anion having a novel structure having a specific polar functional group such as a sulfonate group, a photoresist composition comprising the same, and a method for forming a photoresist pattern.

EP 4 451 061 A1

【FIG. 1】

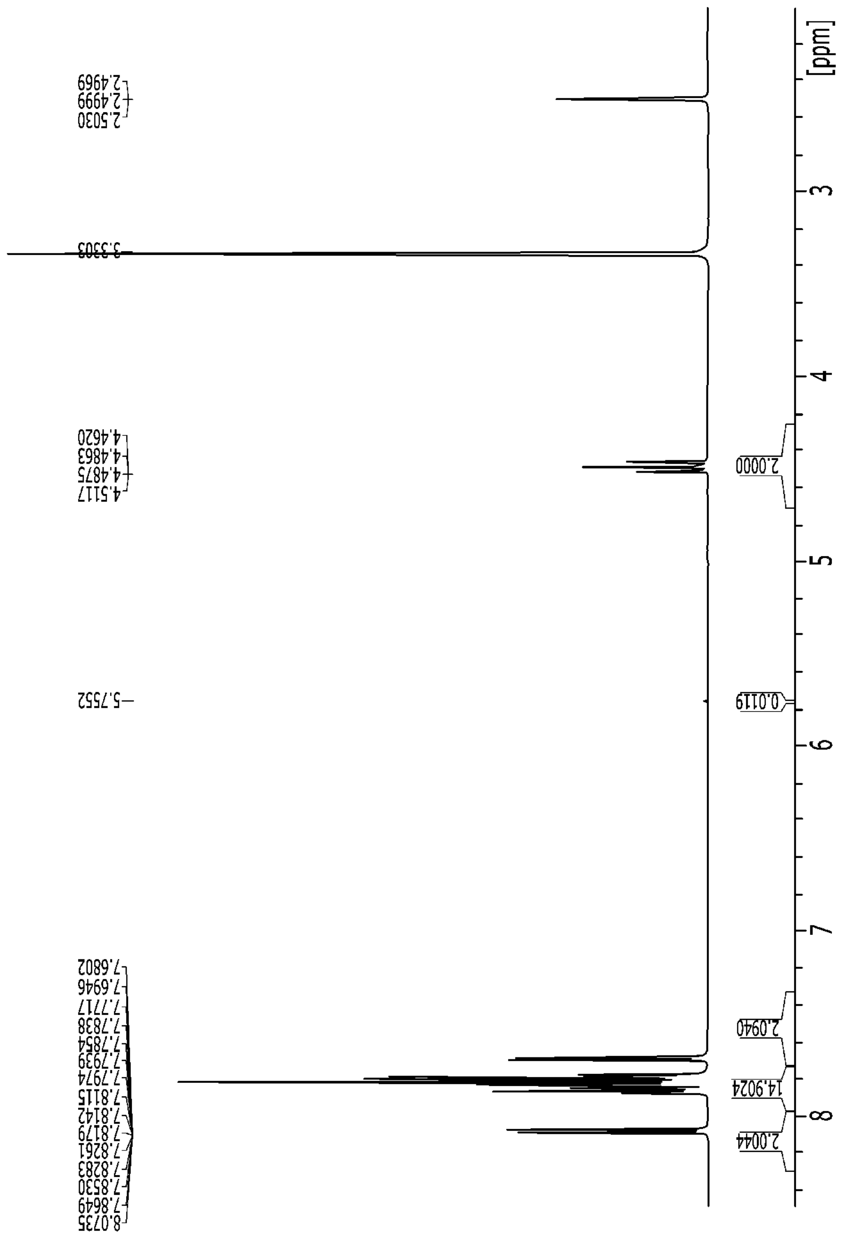
2.4969
2.4999
2.5030
3.3303
4.4620
4.4863
4.4875
4.5117
5.7552
7.6802
7.6946
7.7717
7.7838
7.7854
7.7939
7.7974
7.8115
7.8142
7.8179
7.8261
7.8283
7.8530
7.8649
8.0735
2.0000
0.0119
2.0940
14.9024
2.0044
3
4
5
6
7
8
[ppm]

## Description

### [TECHNICAL FIELD]

The present disclosures relates to a novel photoacid generator, a photoresist composition comprising the same, and a method for forming a photoresist pattern using the same.

### [BACKGROUND ART]

Lithography technology is a technology in which a resist material made of a photosensitive material is coated onto a substrate to form a resist film, and a patterned mask is exposed and developed on this resist film using a light source selected from near ultraviolet (NUV), deep ultraviolet (DUV), electron beam (e-beam), and extreme ultraviolet (EUV), so that a mask pattern is transferred to a resist film to form a pattern. Resist is classified into a positive type, in which an exposed portion dissolves in the developer due to a change in characteristics, and a negative type, in which an exposed portion do not dissolve in the developer.

As light sources with increasingly shorter light source wavelengths were used to miniaturize semiconductor patterns, near-ultraviolet rays (g-line_436nm, i-line_365nm) were used, and currently, deep ultraviolet (KrF_248nm, ArF_193nm) excimer lasers is used, and ArF-immersion and multi-patterning methods using the same are widely used in mass production of semiconductor devices.

The demand for the device integration improvement of semiconductor devices manufactured by applying lithography technology increases year by year, and competition to gain an advantage by securing preemptive technologies is intensifying. In the methods of increasing the number of chips integrated per unit area of the silicon wafer used in semiconductor chip manufacturing, the most powerful method is to minimize the circuit line width (CD_critical dimension). In order to minimize the line width, it is necessary to apply lithography technology having a higher resolution. As mentioned above, the use of a light source with a short wavelength is the most reliable way to increase the resolution of lithography technology. At present, ArF light sources (193 nm) have been mainly used, and in recent years, lithography technology using extreme ultraviolet (EUV, 13.5 nm) wavelengths has begun to be applied to mass production process.

Meanwhile, resist materials are required to have characteristics such as sensitivity to an exposure light source and resolving power to reproduce fine mask patterns. As the resist components that can exhibit such characteristics, a photoacid generator that generates an acid by an exposure process, a polymer resin that changes solubility in an alkali developer due to the action of the generated acid, and an acid diffusion control agent (quencher) that can control the acid diffusion distance may be used, and a solvent that can dissolve respective components may be added to prepare a chemically-amplified photoresist(CAR) composition.

As the base resin of the KrF resist composition, a poly-hydroxystyrene (PHS)-based resin, an acrylic-based resins having acryliester constitution unit, or a resin mixed with them are mainly used. In addition, as the base resin of the ArF resist composition, a methacrylic resin having a methacrylic acid ester constitution unit, which has excellent transparency in an ArF light source, is generally widely used. In the base resin of the EUV resist composition, all monomers used in the base resin for KrF and ArF resists may be used due to the characteristics of a short wavelength light source, so that a wide variety of polymer or copolymer base resins can be selectively used in accordance with their characteristics.

As the photoacid generator used in chemically-amplified photoresist composition, various structures such as onium salts such as sulfonium salt and iodonium salt, diazomethane-based, oxime-based, nitrobenzylsulfonate-based, iminosulfonate-based, and disulfone-based acid generators have been proposed so far, and they may be used alone or in combination of two or more. In the chemically-amplified resist composition, nitrogen-containing organic substances such as amines and amides are used in addition to base resins and acid generators, or furthermore, photosensitive quenchers are used, thereby attempting to improve resist performance by controlling the diffusion of acids generated from photoacid generators.

However, in developing high-resolution resists, fundamental acid diffusion length adjustment is always necessary to overcome the conventional line width roughness (LER/LWR). In addition, in order to improve the acid diffusion rate and length in a resist composition, the anion structure has the greatest influence on the acid strength, diffusion rate, and distance.

However, when using existing resist materials, the line width roughness forming the desired pattern size is still not satisfied.

Therefore, there is still a need to develop resist materials that can adjust the acid diffusion rate and length of new structures capable of securing high contrast and improving process margin during fine pattern formation.

**[DETAILED DESCRIPTION OF THE INVENTION]**

**[Technical Problem]**

It is an object of the present disclosure to provide a novel photoacid generator that can provide a photoresist composition which improves the polarity and thus shortens the acid diffusion length of anions generated during exposure, ensure high contrast, improve marginal resolving power, has low sensitivity and LER/LWR values, and further has a wide process margin while simultaneously satisfying CDU (Critical Dimension uniformity) improvement.
It is another object of the present disclosure to provide a photoresist composition comprising the photoacid generator.
It is yet another object of the present disclosure to provide a method for forming a photoresist pattern.

**[Technical Solution]**

In one aspect of the present disclosure, there is provided a photoacid generator comprising an anion represented by the following Chemical Formula 1, and a cation represented by the following Chemical Formula 2:

[Chemical Formula 1] A-Y-Z-R-EWG

[Chemical Formula 2]

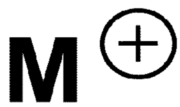

wherein, in Chemical Formulas 1 and 2,
A and Z are the same as or different from each other, and are each independently a polar functional group,
Y is a linker connecting A and Z, which is a divalent organic group,
R represents a chain or cyclic aliphatic hydrocarbon group or a monocyclic or polycyclic aromatic hydrocarbon group,
EWG is an electron withdrawing group substituted for R, and
$M^+$ is an onium ion or a metal ion.

In another aspect of the present disclosure, there is provided a photoresist composition comprising:

the photoacid generator;
a base polymer;
an acid diffusion inhibitor; and
an organic solvent.

In yet another aspect of the present disclosure, there is provided a method for forming a photoresist pattern comprising the steps of:

coating and heating the photoresist composition to form a photoresist
film; exposing the photoresist film; and
developing the exposed photoresist film to form a photoresist pattern.

Embodiments of the present disclosure will be described in more detail below. Terms or words used in the present specification and claims should not be construed as limited to ordinary or dictionary terms, and the present invention should be construed with meanings and concepts that are consistent with the technical idea of the present invention based on the principle that the inventors may appropriately define concepts of the terms to appropriately describe their own invention in the best way.
The terms "comprise," "include", "have", etc. are used herein to specify the presence of stated feature, region, integer, step, action, element and/or component, but do not preclude the presence or addition of other feature, region, integer, step, action, element and/or component.
In the present disclosure, the weight average molecular weight is a polystyrene-converted weight average molecular weight measured by GPC, and the weight average molecular weight was measured by a standard analysis

method of the gel permeation chromatography(GPC) system using Waters Alliance e2695 Separation Module.

In the present disclosure, the term "polymer" may be used interchangeably with the term "macromolecule" or "high molecule".

In the present disclosure, examples of substituents are explained below, but are not limited thereto.

In the present disclosure, the term "substituted" means that another atom or functional group is bonded in place of a hydrogen atom or carbon atom in a compound. The position to be substituted is not limited as long as it is a position where a hydrogen atom or a carbon atom is substituted, that is, a position where a substituent can be substituted. When two or more substituents are substituted, the two or more substituents may be the same as or different from each other.

In the present disclosure, the term "substituted or unsubstituted" means being unsubstituted or substituted with one or more substituent groups selected from the group consisting of halogen; a cyano group; a nitro group; a hydroxy group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyl group; an aryloxy group; an alkylthioxy group; an arylthioxy group; an alkylsulfoxy group; an arylsulfoxy group; a silyl group; and a boron group; or being unsubstituted or substituted with a substituent group to which two or more substituent groups of the above-exemplified substituent groups are linked. For example, "a substituent group in which two or more substituents are linked" may be a biphenyl group. Namely, a biphenyl group may be an aryl group, or it may be interpreted as a substituent group in which two phenyl groups are linked.

In the present disclosure, the notation

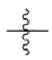 or 

means a bond linked to another substituent group.

In the present disclosure, examples of a halogen group include fluorine, chlorine, bromine, or iodine.

In the present disclosure, the carbon number of an imide group is not particularly limited, but is preferably 1 to 30. Specifically, the imide group may be a compound having the following structural formulas, but is not limited thereto.

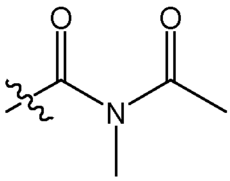
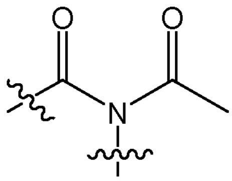
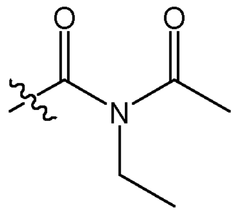

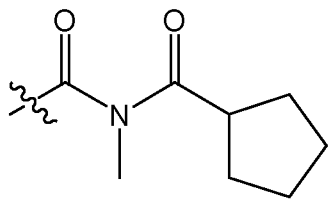
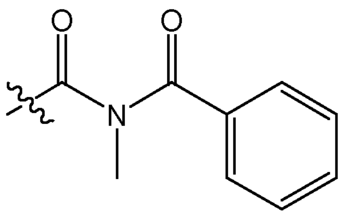

In the present disclosure, an amide group may have a structure in which nitrogen of the amide group may be substituted with hydrogen, a straight-chain, branched-chain, or cyclic alkyl group having 1 to 30 carbon atoms, or an aryl group having 6 to 30 carbon atoms. Specifically, the amide group may be a compound having the following structural formula, but is not limited thereto.

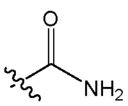
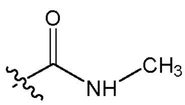
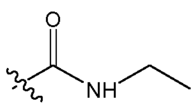
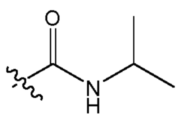

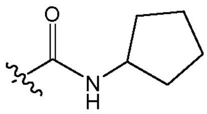 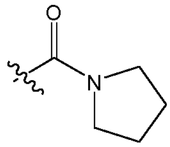 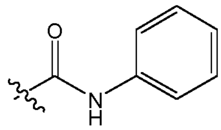 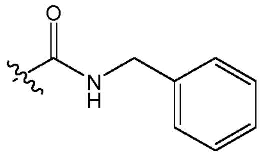

In the present disclosure, the carbon number of a carbonyl group is not particularly limited, but is preferably 1 to 30. Specifically, the carbonyl group may be a compound having the following structural formulas, but is not limited thereto.

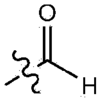 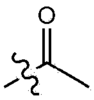 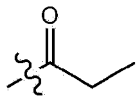 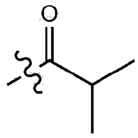

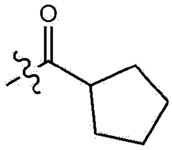 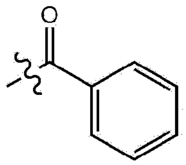 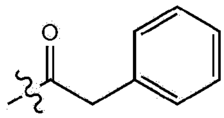

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a compound having the following structural formulas, but is not limited thereto.

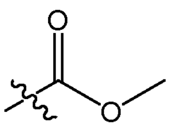 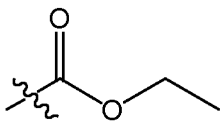 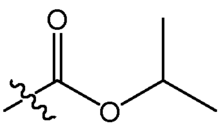 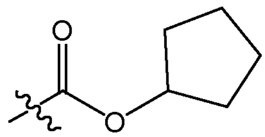

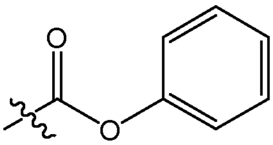 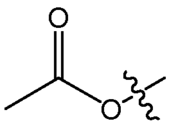 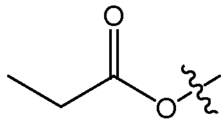 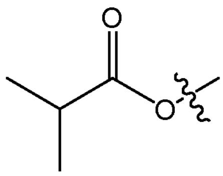

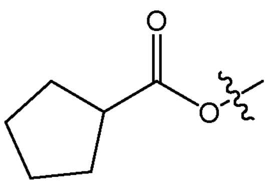 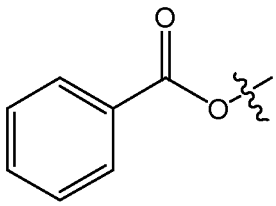

In the present disclosure, a sulfonamide group may be $-SO_2NR'R''$, wherein the R' and R" are the same as or different from each other, and may be each independently selected from the group consisting of hydrogen; deuterium; halogen; a nitrile group; a substituted or unsubstituted monocyclic or polycyclic cycloalkyl group having 3 to 30 carbon atoms; a substituted or unsubstituted straight-chain or branched-chain alkyl group having 1 to 30 carbon atoms; a substituted or unsubstituted monocyclic or polycyclic aryl group having 6 to 30 carbon atoms; and a substituted or unsubstituted monocyclic or polycyclic heteroaryl group having 2 to 30 carbon atoms.

In the present disclosure, an alkyl group may be straight-chain or branched-chain, and the carbon number of the straight-chain alkyl group is not particularly limited, but may be 1 to 20. Also, the carbon number of the branched-chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto. The alkyl group may be substituted or unsubstituted, and if substituted, examples of the substituent are as described above.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto. The cycloalkyl group may be substituted or unsubstituted, and if substituted, examples of the substituent are as described above.

In the present disclosure, an alkoxy group is a functional group in which the above-mentioned alkyl group is bonded to one end of an ether group(-O-), and the description of the alkyl group as defined may be applied, except that each of these are functional groups is bonded to an ether group (-O-). For example, the alkoxy group may be straight chain, branched chain, or cyclic chain. The carbon number of the alkoxy group is not particularly limited, but may be 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, cycloheptoxy, benzyloxy, p-methylbenzyloxy, and the like, but are not limited thereto. The alkoxy group may be substituted or unsubstituted, and if substituted, examples of the substituent are as described above.

In the present disclosure, an amine group may be selected from the group consisting of $-NH_2$, a monoalkylamine group, a dialkylamine group, an N-alkylarylamine group, a monoarylamine group, a diarylamine group, an N-arylheteroarylamine group, an N-alkylheteroarylamine group, a monoheteroarylamine group and a diheteroarylamine, and the carbon number thereof is not particularly limited, but may be 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a ditolylamine group, an N-phenylbiphenylamine group, an N-phenylnaphthylamine group, an N-biphenylnaphthylamine group, a ditolylamine group, an N-phenyltolylamine group, a triphenylamine group, an N-naphthylfluorenylamine group, an N-phenylphenanthrenylamine group, an N-biphenylphenanthrenylamine group, an N-phenylfluorenylamine group, an N-phenylterphenylamine group, an N-phenanthrenylfluorenylamine group, an N-biphenylfluorenylamine group, and the like, but are not limited thereto. The amine group may be substituted or unsubstituted, and if substituted, examples of the substituent are as described above.

In the present disclosure, an N-alkylarylamine group means an amine group in which N of the amine group is substituted with an alkyl group and an aryl group.

In the present disclosure, an N-arylheteroarylamine group means an amine group in which N of the amine group is substituted with an aryl group and a heteroaryl group.

In the present disclosure, an N-alkylheteroarylamine group means an amine group in which N of the amine group is substituted with an alkyl group and a heteroarylamine group.

In the present disclosure, an alkyl group in the monoalkylamine group, dialkylamine group, N-arylalkylamine group, alkylthioxy group, alkylsulfoxy group, and N-alkylheteroarylamine group are the same as examples of the alkyl group as defined above. Specifically, the alkylthioxy group includes a methylthioxy group, an ethylthioxy group, a tert-butylthioxy group, a hexylthioxy group, an octylthioxy group, and the like, and the alkyl sulfoxy group includes a mesyl group, an ethyl sulfoxy group, a propyl sulfoxy group, a butyl sulfoxy group, and the like, but are not limited thereto.

In the present disclosure, an alkenyl group may be straight-chain or branched-chain, and the carbon number thereof is not particularly limited, but may be 2 to 40. The description of the alkyl group as defined above may be applied except that the alkenyl group includes at least one unsaturated bond (double bond or triple bond) in the functional group. According to one embodiment, the carbon number of the alkenyl group is 2 to 20. According to another embodiment, the carbon number of the alkenyl group is 2 to 10. According to another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto. The alkenyl group may be substituted or unsubstituted, and if substituted, examples of the substituent are as described above.

In the present disclosure, a cycloalkenyl group may have 3 to 60 carbon atoms. According to one embodiment, the carbon number of the cycloalkenyl group is 3 to 30. According to another embodiment, the carbon number of the

cycloalkenyl group is 3 to 20. According to another embodiment, the carbon number of the cycloalkenyl group is 3 to 6. The description of the cycloalkyl group as defined above may be applied except that the cycloalkenyl group includes at least one unsaturated bond (double bond or triple bond) in the functional group. The cycloalkenyl group may be substituted or unsubstituted, and if substituted, examples of the substituent are as described above.

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto. The silyl group may be substituted or unsubstituted, and if substituted, examples of the substituent are as described above.

In the present disclosure, a boron group specifically includes a trimethyl boron group, a triethyl boron group, a t-butyldimethyl boron group, a triphenyl boron group, and a phenyl boron group, but is not limited thereto. The boron group may be substituted or unsubstituted, and if substituted, examples of the substituent are as described above.

In the present disclosure, an aryl group is not particularly limited, but may have 6 to 60 carbon atoms, and may be a monocyclic aryl group or a polycyclic aryl group. According to one embodiment, the carbon number of the aryl group is 6 to 30. According to another embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto. The aryl group may be substituted or unsubstituted, and if substituted, examples of the substituent are as described above.

In the present disclosure, an aryl group in the monoarylamine group, diarylamine group, aryloxy group, arylthioxy group, arylsulfoxy group, N-arylalkylamine group, N-arylheteroarylamine group and arylphosphine group is the same as the example of the aryl group as defined above. Specifically, the the aryloxy group includes phenoxy group, p-toryloxy group, m-toryloxy group, 3,5-dimethyl-phenoxy group, 2,4,6-trimethylphenoxy group, p-tert-butylphenoxy group, 3-biphenyloxy group, 4-biphenyloxy group, 1-naphthyloxy group, 2-naphthyloxy group, 4-methyl-1-naphthyloxy group, 5-methyl-2-naphthyloxy group, 1-anthryloxy group, 2-anthryloxy group, 9-anthryloxy group, 1-phenanthryloxy group, 3-phenanthryloxy group, 9-phenanthryloxy group, or the like, the arylthioxy group includes phenylthioxy group, 2-methylphenylthioxy group, and 4-tert-butylphenylthioxy group, or the like, and the aryl sulfoxy group includes benzene sulfoxy group and p-toluene sulfoxy group, or the like, but are not limited thereto.

In the present disclosure, a heterocyclic group is a heterocyclic group containing one or more of O, N, Se and S as a heteroatom, and the carbon number thereof is not particularly limited, but may be 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, a triazole group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, a dibenzofuranyl group, and the like, but are not limited thereto.

In the present disclosure, a heteroaryl group includes at least one heteroatom (i.e. atom other than carbon). Specifically, the heteroatom may include at least one atom selected from the group consisting of O, N, Se, and S. The carbon number thereof is not particularly limited, but may be 2 to 60 carbon atoms. The heteroaryl group may be monocyclic or polycyclic. Examples of the heteroaryl group include a thiophene group, a furanyl group, a pyrrole group, an imidazolyl group, a thiazolyl group, an oxazolyl group, an oxadiazolyl group, a pyridyl group, a bipyridyl group, a pyrimidyl group, a triazinyl group, a triazolyl group, an acridyl group, a pyridazinyl group, a pyrazinyl group, a quinolinyl group, a quinazolinyl group, a quinoxalinyl group, a phthalazinyl group, a pyrido pyrimidyl group, a pyrido pyrazinyl group, a pyrazino pyrazinyl group, an isoquinolinyl group, an indolyl group, a carbazolyl group, a benzoxazolyl group, a benzimidazolyl group, a benzothiazolyl group, a benzocarbazolyl group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, an aziridyl group, an azaindolyl group, an isoindolyl group, an indazolyl group, a purine group, a pteridine group, a beta-carbonyl group, a naphthyridine group, a terpyridyl group, a phenazinyl group, an imidazopyridyl group, a pyropyridyl group, an azepine group, a pyrazolyl group, a dibenzofuranyl group, and the like, but are not limited thereto. The heteroaryl group may be substituted or unsubstituted, and if substituted, examples of the substituent are as described above.

In the present disclosure, examples of the heteroaryl group in the monoheteroarylamine group, diheteroarylamine group, N-arylheteroarylamine group, and N-alkylheteroarylamine group may be selected from examples of the heteroaryl group as defined above.

Now, a novel photoacid generator, a photoresist composition comprising the same, and a method for forming a photoresist pattern using the photoresist composition according to one embodiment of the present disclosures will be described in more detail.

**Novel photoacid generator**

According to one embodiment of the present disclosure, there can be provided a photoacid generator comprising an anion represented by the following Chemical Formula 1, and a cation represented by the following Chemical Formula 2:

[Chemical Formula 1] A-Y-Z-R-EWG

[Chemical Formula 1]

[Chemical Formula 2]

$$M^{\oplus}$$

wherein, in Chemical Formulas 1 and 2,
A and Z are the same as or different from each other, and are each independently a polar functional group,
Y is a linker connecting A and Z, which is a divalent organic group,
R represents a chain or cyclic aliphatic hydrocarbon group or a monocyclic or polycyclic aromatic hydrocarbon group,
EWG is an electron withdrawing group substituted for R, and
$M^+$ is an onium ion or a metal ion.

The present inventors have found through experiments that, when a photoacid generator has a substituted structure containing a polarity group such as a sulfonyloxy group ($-SO_3-$) or a carbonyloxy group (-(C=O)O-) in the anion structure represented by Chemical Formula 1, more polarity can be imparted than before and thus, an acid diffusion length of the anion generated during exposure is shortened, a high contrast is secured, a marginal resolving power is improved, a low sensitivity and an LER value are improved, and a wide process margin is obtained, and completed the present disclosure.

In particular, when the anion of Chemical Formula 1 contains an aromatic sulfonyloxy group substituted with an electron withdrawing group(EWG), it can improve exposure sensitivity and also secure high contrast between an exposed portion and a non-exposed portion compared to a case where it does not, thereby obtaining a resist composition having an improved resolving power, a low line width roughness value, and a process margin improvement capacity.

Therefore, the present disclosure can provide a novel photoacid generator that includes a specific anion part structure, has improved acid strength, and anion diffusion rate and diffusion length compared to conventional ones, and thus can be used to manufacture a high resolution resist.

According to the photoacid generator of one embodiment of the disclosure, in the anion of Chemical Formula 1, the A may be either a sulfonate group ($-SO_3^-$) or a carbonate group ($-CO_3^{2-}$). Specifically, A in Chemical Formula 1 may be a sulfonate group ($-SO_3^-$), in which case the polarity of the sulfonate group is relatively higher than that of the carbonate group, which may shorten the diffusion distance of the anion. That is, the sulfonate group can provide the effect of shortening the acid diffusion distance, and thus can improve the resolving power.

In the anion of Chemical Formula 1, the Y is a linker connecting A and Z, which may be a divalent organic group, and may specifically mean a divalent hydrocarbon radical in which at least one halogen element is substituted.

The Y may be any one of an alkylene groups having 1 to 10 carbon atoms substituted with an atomic group containing at least one halogen element. The halogen element may be F. Therefore, according to one embodiment, in the anion of Chemical Formula 1, the Y may be any one of an alkylene group having 1 to 10 carbon atoms substituted with an atomic group containing at least one F. As the alkylene chain that is not substituted by F from the Y becomes longer, the synthesis is more difficult, crystallization at the time of purification is more difficult and thus removal of impurities is more difficult, which thus makes it difficult to secure purity.

That is, in the anion structure represented by Chemical Formula 1, when the polar functional groups A and Z are bonded via Y, the Y can lower the acidity when the atomic group includes at least one halogen element. Specifically, the Y may be any one of an alkylene group having 2 to 5 carbon atoms substituted with 2 to 4 F. At this time, as the alkylene chain that is not substituted with F is shorter, it may be more advantageous for the synthesis and purification efficiency.

When two Fs are included in the Y, the inclusion thereof on the left side of the carbon chain may be advantageous for the synthesis.

In addition, in the anion of Chemical Formula 1, the Z may be either a sulfonyloxy group($-SO_3^-$) or a carbonyloxy

group(-C(=O)O-). Specifically, Z in Chemical Formula 1 may be a sulfonyloxy group(-$SO_3^-$), in which case the polarity of the sulfonyloxy group is relatively higher than that of the carbonyloxy group, which can further shorten the diffusion distance of the anion.

Therefore, when the A is a sulfonate group and the Z is a sulfonyloxy group, it is possible to improve the contrast more effectively.

In addition, in the anion of Chemical Formula 1, the R may be a chain or cyclic aliphatic hydrocarbon group or a monocyclic or polycyclic aromatic hydrocarbon group. The R contains an electron withdrawing group (EWG) as a substituent. As the number of electron withdrawing groups is greater, it is possible to provide more advantageous effect for sensitivity.

The R may be either a cyclic aliphatic hydrocarbon group and an aromatic hydrocarbon group, specifically a monocyclic or polycyclic aromatic hydrocarbon group, and more specifically a monocyclic aromatic hydrocarbon having 6 to 30 carbon atoms. That is, when the R is a cyclic aliphatic hydrocarbon group or a monocyclic or polycyclic aromatic hydrocarbon group, the absorption efficiency can be increased compared to a case where R is a chain hydrocarbon group. Further, when R is a monocyclic aromatic group, there is an advantage in that solubility is increased. Therefore, it may be advantageous that R contains a monocyclic aromatic group in terms of absorption efficiency and solubility.

At this time, the chain or cyclic aliphatic hydrocarbon group of R is an alkyl group, an alkenyl group, a cycloalkyl group, or a cycloalkenyl group, which is substituted with one or more electron withdrawing groups (EWG), the alkyl group and the alkenyl group are each independently a straight chain or branched chain group having 1 to 30 carbon atoms, and the cycloalkyl group or the cycloalkenyl group may each independently be a monocyclic or polycyclic group having 3 to 30 carbon atoms.

In addition, in the anion of Chemical Formula 1, the EWG is an electron withdrawing group that acts as an activity reducing group substituted via -R.

The EWG is linked, substituted and linked to a sulfonyloxy group via R, thereby exhibiting exposure sensitivity improvement more effectively and securing high contrast between an exposed portion and an non-exposed portion, which may improve the marginal resolving power.

In particular, when the anion of Chemical Formula 1 contains an aromatic sulfonyloxy group substituted with an electron withdrawing group, it can improve exposure sensitivity and also secure high contrast between an exposed portion and a non-exposed portion compared to a case where it does not, thereby obtaining a resist composition having an improved resolving power, a low line width roughness value, and a process margin improvement capacity.

Specifically, the EWG may be one or more electron withdrawing groups selected from the group consisting of: a halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyl group; an aryloxy group; an alkylthioxy group; an arylthioxy group; an alkyl sulphoxy group; an aryl sulfoxy group; a silyl group; a boron group; and a cycloalkyl group.

Specifically, the EWG may be one or more electron withdrawing groups selected from a nitro group, a halogen, a fluoroalkyl group, a cyano group, and a sulfonyl group. The nitro group, the halogen, the fluoroalkyl group, the cyano group and the sulfonyl group have higher EUV light source absorption efficiency than the electron withdrawing groups exemplified above. Among them, the EWG may be any one selected from a halogen group, a sulfonyl group, and a nitro group, and the halogen group may be most excellent in EUV light source absorption efficiency. That is, the EUV light source absorption efficiency can be further improved in the order of a nitro group, a sulfonyl group and a halogen group.

Therefore, the EWG may be a halogen group or a sulfonyl group, and more specifically, may be a sulfonyl group. The halogen may be F or I, and according to a preferred embodiment, it may be I.

According to one embodiment of the disclosure, when R has an aromatic structure and also includes an electron withdrawing group (EWG) such as a halogen element, particularly iodine, it can exhibit higher light absorption ability for the EUV light source, thereby further maximizing resist performance improvement.

Such an EWG may have a cycloalkyl group which is unsubstituted or substituted with at least one heteroatom. Further, the EWG may have a cycloalkyl group substituted with one or more heteroatoms having 3 to 30 carbon atoms which is substituted with a sulfonyl group, or a cycloalkyl group substituted with one or more heteroatoms having 4 to 10 carbon atoms which is substituted with a sulfonyl group. The heteroatom may be nitrogen or oxygen. For example, the EWG may have any one of the following structures.

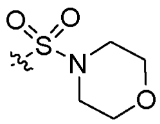

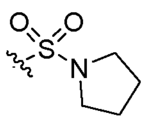

More specifically, in the structure of -R-EWG of Chemical Formula 1, the chain or cyclic aliphatic hydrocarbon or aromatic hydrocarbon of the R may have an electron withdrawing group in which at least one hydrogen atom of R is

substituted.

Therefore, -R-EWG in Chemical Formula 1 may be an alkyl group(R) having 1 to 30 carbon atoms in which at least one hydrogen atom of R of the chain aliphatic hydrocarbon is substituted with a halogen(EWG). Specific examples of the alkyl group having 1 to 30 carbon atoms are not particularly limited, but one example thereof may include one of methyl, ethyl, or propyl. Therefore, specific examples of the alkyl group having 1 to 30 carbon atoms in which at least one hydrogen atom is substituted with a halogen, which is the -R-EWG, is not particularly limited, but one example thereof may include trifluoromethyl in which the methyl group is substituted with a halogen.

Further, in the structure of -R-EWG of Chemical Formula 1, the cyclic hydrocarbon may be a cycloalkyl group(R) having 3 to 30 carbon atoms in which at least one hydrogen atom of R is substituted with a halogen(EWG). Specific examples of the cycloalkyl group having 3 to 30 carbon atoms in which the hydrogen atom is substituted, which is the -R-EWG, are not particularly limited, but one example thereof may include 4-fluorocyclohexyl or the like.

Further, in the structure of -R-EWG of Chemical Formula 1, the aromatic hydrocarbon is a monocyclic aryl group(R) having 6 to 30 carbon atoms in which at least one hydrogen atom is substituted with any one EWG selected from a nitro group, a halogen, a fluoroalkyl group, a cyano group, and a sulfonyl group.

Specific examples of the -R-EWG structure are not particularly limited, but as an example, any one of 4-trifluoromethylphenyl, 4-fluorophenyl, 2,5-difluorophenyl, 2,4-difluorophenyl, 2,3-difluorophenyl, 4-(trifluoromethyl)phenyl, 1,2,3,4,5-pentafluorophenyl, 4-bromophenyl, 4-iodophenyl, 3,5-diiodophenyl, 4-cyanophenyl, 4-nitrophenyl, and 4-sulfonylphenyl may be mentioned.

Specifically, in Chemical Formula 1, when R is an aryl group having 6 to 30 carbon atoms in which 2 or more and 6 or less hydrogen atoms are substituted with a halogen, it is advantageous in terms of improving absorption efficiency and solubility.

An example of -R-EWG in Chemical Formula 1 may be one of methyl, ethyl, propyl, trifluoromethyl, cyclohexyl, adamantyl, 4-trifluoromethylphenyl, 4-fluorophenyl, 2,5-difluorophenyl, 2,4-difluorophenyl, 2,3-difluorophenyl, 4-(trifluoromethyl)phenyl, 1,2,3,4,5-pentafluorophenyl, 4-bromophenyl, 4-iodophenyl, 3,5-diiodophenyl, 4-cyanophenyl, or 4-nitrophenyl, 4-sulfonylphenyl, and more specifically, it may be one of 4-fluorophenyl, 4-bromophenyl, 4-iodophenyl, 2,4-difluorophenyl, 2,5-difluorophenyl, 2,3-difluorophenyl, 4-(trifluoromethyl)phenyl, or 1,2,3,4,5-pentafluorophenyl. However, the -R-EWG is not limited thereto, and may include structures included in examples of anions described below.

According to one embodiment of the disclosure, the photoacid generator may be a compound represented by the following Chemical Formula 3.

[Chemical Formula 3]

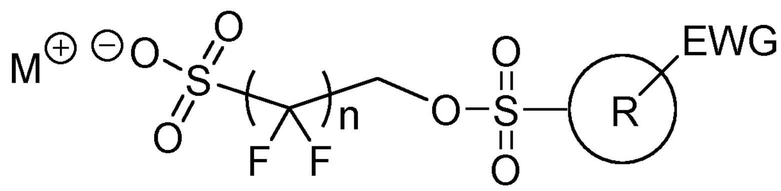

wherein, in Chemical Formula 3,
n is an integer of 1 to 10, and
R, EWG and $M^+$ are as defined above.

That is, in the case of Chemical Formula 3, the anionic portion of Chemical Formula 1 can be divided into A-Y-Z-R-EWG, as shown in the following Structural Formula 3-1.

[Chemical Formula 3-1]

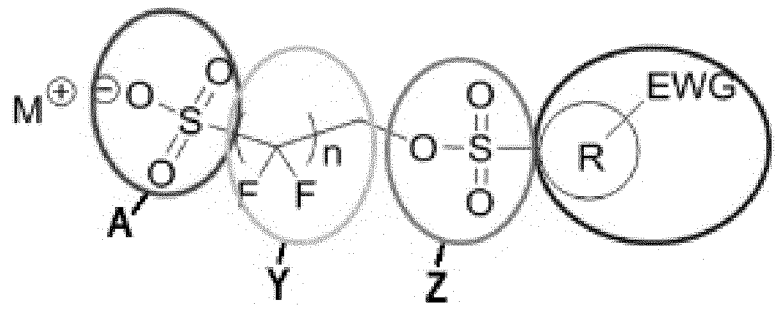

Specifically, as shown in Chemical Formulas 3 and 3-1, in Chemical Formula 1, A is a sulfonate group ($-SO_3^-$), Y is an atomic group-substituted alkylene containing two or more F, and Z may be a sulfonyloxy group ($-SO_3^-$).

In addition, in Chemical Formulas 3 and 3-1, R is a monocyclic or polycyclic aromatic hydrocarbon group, and EWG as the electron withdrawing group may be any one or more selected from a halogen group, a nitro group, a halogen, a fluoroalkyl group, a cyano group, and a sulfonyl group. More specifically, the R is a monocyclic aromatic hydrocarbon group, and EWG as the electron withdrawing group may be a halogen group.

In such a case, an aromatic group (R) substituted by an electron withdrawing group(EWG) is substituted in a sulfonyloxy group of Z, thereby increasing electron beam and EUV photon absorption, which can generate more secondary electrons in the resist thin film and improve sensitivity. Further, as mentioned above, the aromatic structure R can maximize the resist performance improvement when it contains a halogen element, particularly iodine, having higher light absorption ability for EUV light source.

Further, EWG as the electron withdrawing group may have a cycloalkyl group which is substituted with at least one heteroatom.

Further, n may be an integer of 1 to 2.

Specific examples of the anion represented by Chemical Formula 1 are not particularly limited, but as an example, it may be any one selected from the group consisting of:

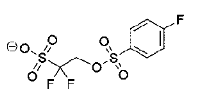 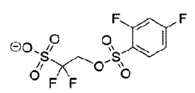 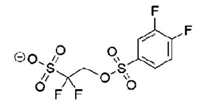 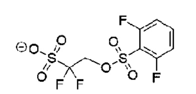 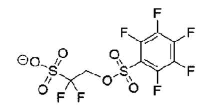

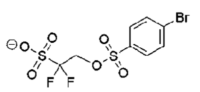 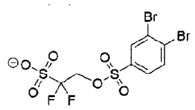 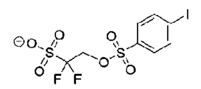 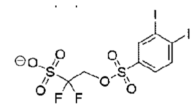 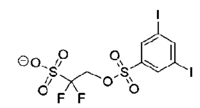

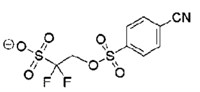 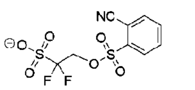 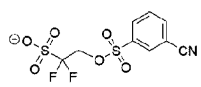 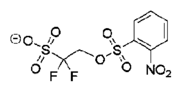 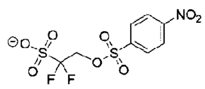

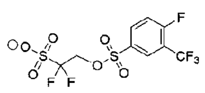 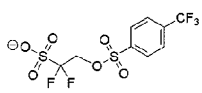 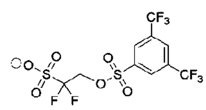 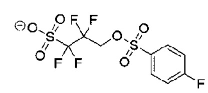 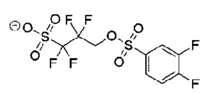

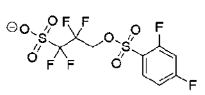 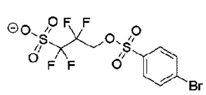 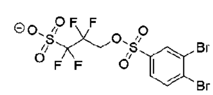 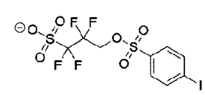 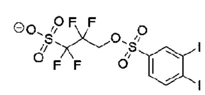

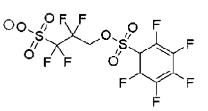 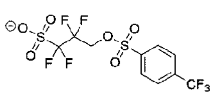 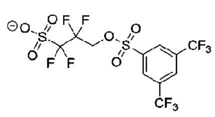 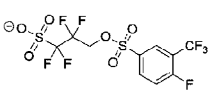 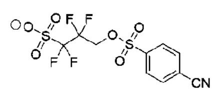

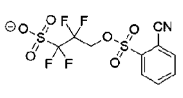 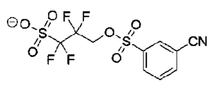 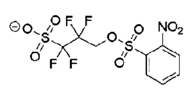 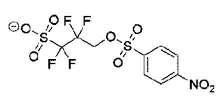 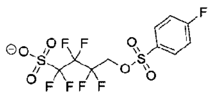

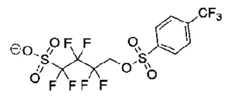 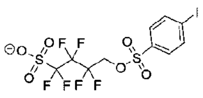 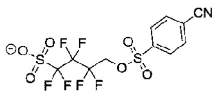 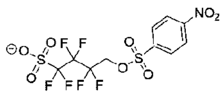 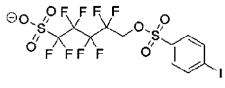

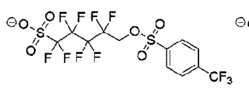 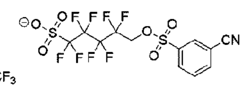 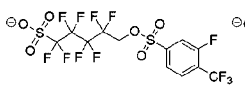 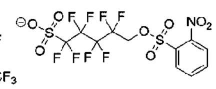 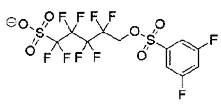

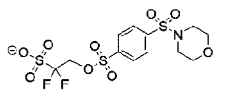 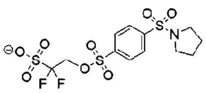 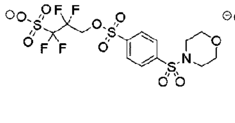 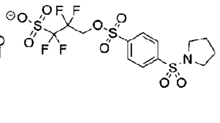 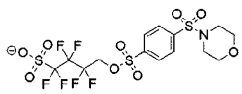

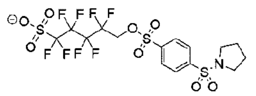 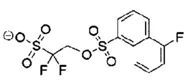 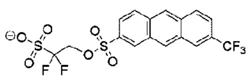 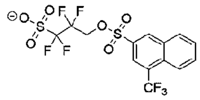 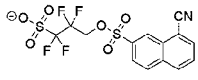

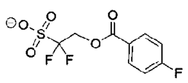 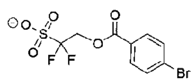 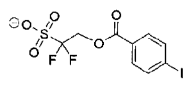 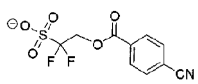 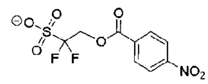

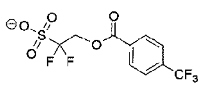 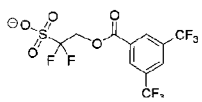 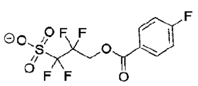 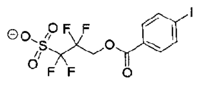 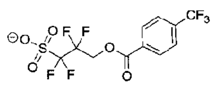

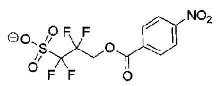 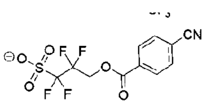 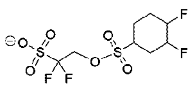 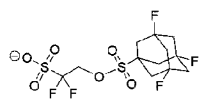 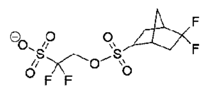

As mentioned above, the photoacid generator having a novel structure containing an anion having a specific polar functional group of Chemical Formula 1 has a sulfonyloxy group in which the anion includes a substituent having an electron withdrawing group, thereby improving the polarity, shortening the acid diffusion length, which may secure high contrast resulting therefrom and improve the marginal resolving power. In addition, the photoacid generator can have a low LER/LWR value and simultaneously satisfy CDU improvement, and thus can be used as an effective resist material to provide a photoresist composition having a wide process margin.

In particular, when R-EWG including an unsaturated aromatic group having an electron withdrawing group is selected and substituted in Chemical Formula 1, it is possible to increase electron beam and EUV photon absorption, which may generate more secondary electrons in the resist thin film and improve sensitivity. Further, it contains a halogen element having a higher light absorption ability for the EUV light source, thereby maximizing the resist performance improvement.

Meanwhile, in Chemical Formula 2, $M^+$ may be either an onium ion or a metal ion. Specifically, in Chemical Formula 2, $M^+$ may be one of an onium ion, a sulfonium ion, an iodonium ion, or an ammonium ion.

Specific examples of the cation represented by Chemical Formula 2 are not particularly limited, but as an example, it may be any one selected from the group consisting of:

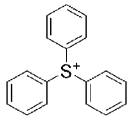 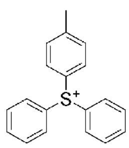 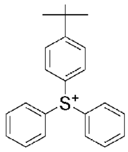 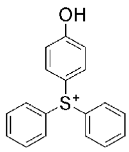 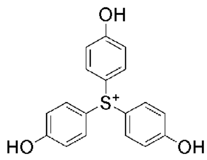 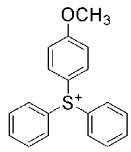

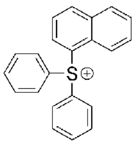 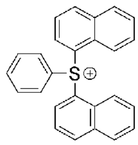 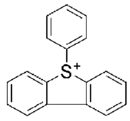 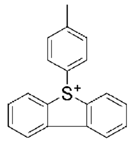 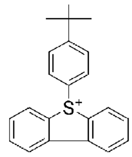 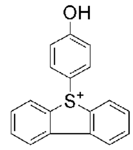

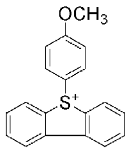 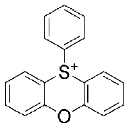 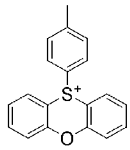 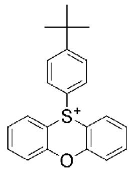 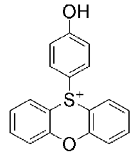 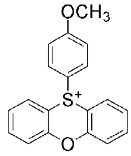

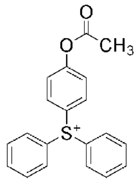 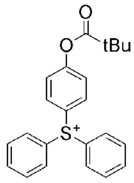 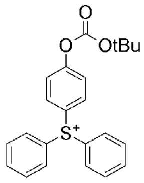 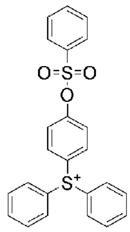 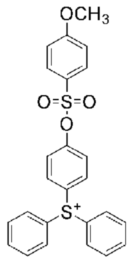 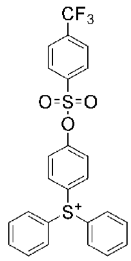

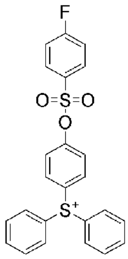 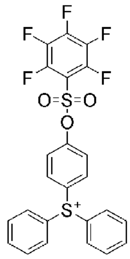 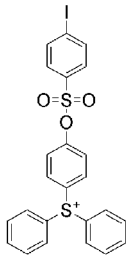 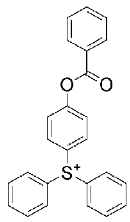 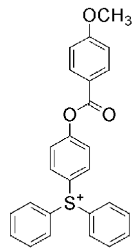 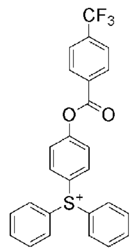

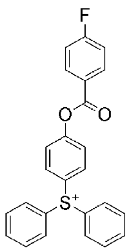 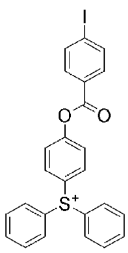 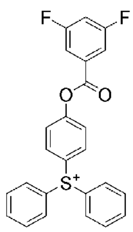 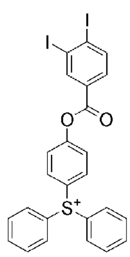 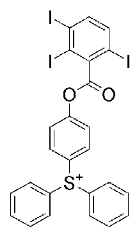 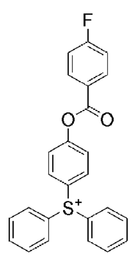

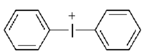 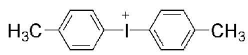 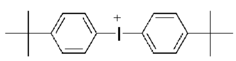 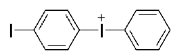

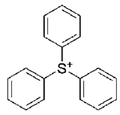 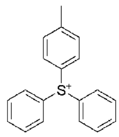 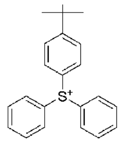 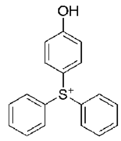 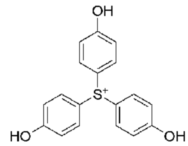 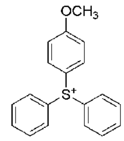

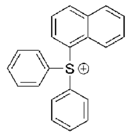 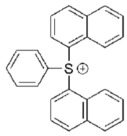 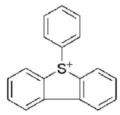 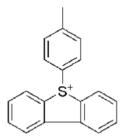 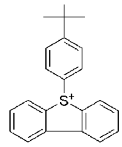 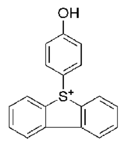

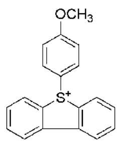 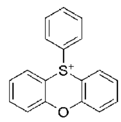 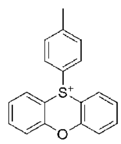 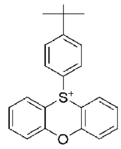 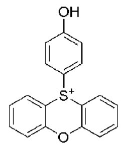 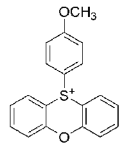

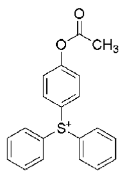 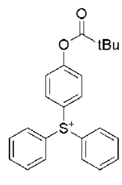 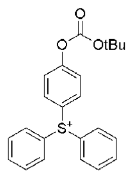 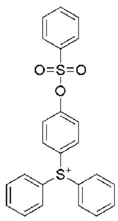 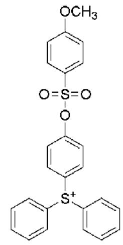 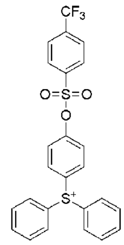

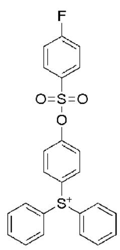 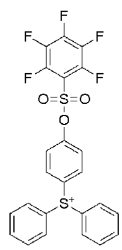 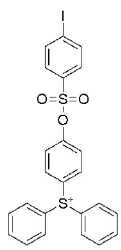 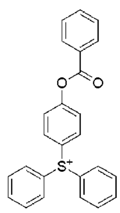 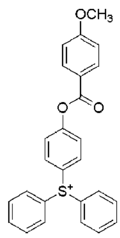 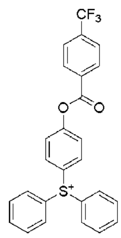

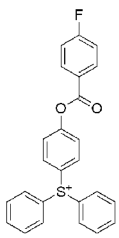 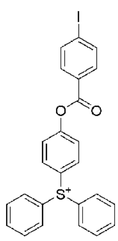 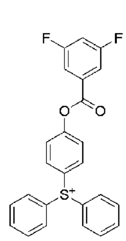 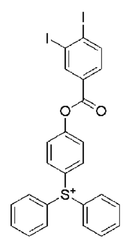 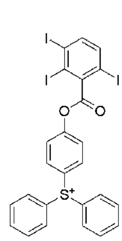 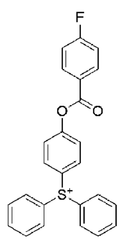

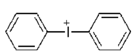 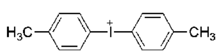 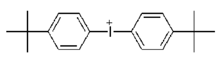 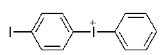

The specific example of the method for synthesizing the salt compound is not particularly limited, but as an example, it can be synthesized according to the following Reaction Scheme 1.

[Reaction Scheme 1]

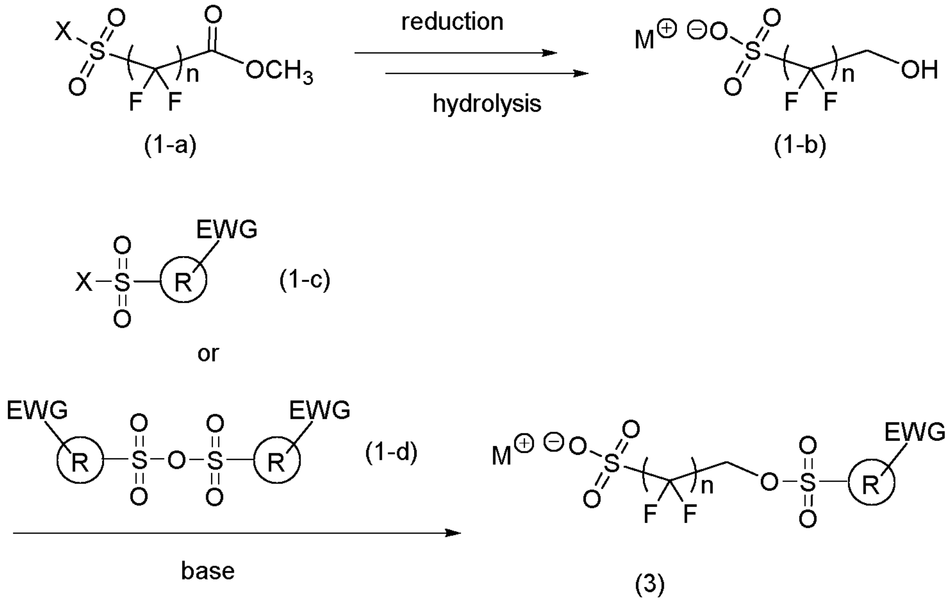

(1-a) (1-b)
(1-c)
or
(1-d)
(3)

in Reaction Scheme 1, R, M, EWG, and n are each the same as those defined in the onium salt-containing compound which includes Chemical Formula 1 and Chemical Formula 2, X is a halogen element as a counter anion, and base is an organic base.

First, Compound (1-a) may be subjected to a reduction reaction and a hydrolysis reaction to obtain Compound (1-b). Then, Compound (1-b) may be reacted again with Compound (1-c) or Compound (1-d) under basic conditions to obtain Compound (3).

In the reduction reaction for obtain the Compound (1-b), an alcohol group can be obtained from the ester group using a reducing agent that generates an appropriate hydrogen ion. The reducing agent reagent is not particularly limited, and generally, sodium borohydride($NaBH_4$), lithium aluminum hydride($LiAlH_4$), diisobutyl aluminum hydride (DIBAH), and the like may be used, and the amount used may be 1 to 1.5 moles relative to 1 mole of Compound (1-a).

In addition, the compound and the method used to provide the cation of Compound (1-b) through the hydrolysis are not particularly limited, and it can be obtained by dissolving the reduction reaction product of Compound (1-a) in an appropriate organic solvent and water using a suitable onium ion or metal ion-containing compound well known in this field, reacting them and then stirring the reaction mixture so that the organic layer and the aqueous layer are well mixed.

As a method for the subsequent hydrolysis reaction after the reduction reaction, it may be obtained through reaction with an appropriate alkali metal hydroxide. The alkali metal hydroxide is not particularly limited, but generally sodium hydroxide, potassium hydroxide, and the like may be used, and the amount used may be 1 to 1.5 moles relative to 1 mole of Compound (1-a).

As the organic solvent in the above reaction, a solvent capable of easily dissolving the product (1-b) and the onium salt-containing compound, such as dichloromethane, chloroform, and acetonitrile may be used, and the amount of the organic solvent used may be 5 parts by weight or more and 20 parts by weight or less for each compound.

The amount of Compounds (1-c) and (1-d) used in the above reaction can usually be 1~ 2 moles relative to 1 mole of Compound (1-b). The reaction time varies depending on reactivity or reaction concentration, but generally may be 10~24 hours, and the reaction temperature may be 0°C~25°C.

After the reaction is completed, the aqueous layer and the organic layer are separated, and the reaction product dissolved in the organic layer can be purified by a method selected from conventionally known purification methods such as solvent extraction, concentration, chromatography, crystallization, and recrystallization, either alone or in combination. This method can also be applied to purify not only the final compound (3) but also Compound (1-d), Compound (1-g), and Compound (1-h) synthesized in each step.

## Photoresist composition

On the other hand, according to another embodiment of the present disclosure, there can be provided a photoresist composition comprising the novel photoacid generator of one embodiment; a base polymer; an acid diffusion inhibitor; and an organic solvent.

The details regarding the photoacid generator includes the contents described above regarding the one embodiment. The content of the photoacid generator may be 1 part by weight or more and 90 parts by weight or less, preferably 5 parts by weight or more and 80 parts by weight or less, and more preferably 10 parts by weight or more and 70 parts by weight or less with respect to 100 parts by weight of the base polymer. If the content of the photoacid generator is 1 part by weight or less, the content is too small, and the acid generation amount of an anion is insufficient, the sensitivity to light is weakened, and not only resolving power but also resist pattern performance deterioration occur. If the content of the photoacid generator exceeds 90 parts by weight, the sensitivity to light increases, but a large amount of acid is generated in the exposed portion, making it difficult to adjust the acid diffusion distance, and there is a risk that the pattern becomes defective. In addition, in terms of improving resolving power and resist pattern formation, and further optimizing the increase in the acid generation amount of the anion and the sensitivity to light, the content of the photoacid generator may be 5 parts by weight or more and 80 parts by weight or less, or 10 parts by weight or more and 70 parts by weight or less.

Meanwhile, the resist composition of the present disclosure includes the onium salt photoacid generator compound and a polymer resin component whose solubility may be changed in a developer by the action of an acid.

The base polymer has the property of changing solubility in a developer due to the action of an acid, and alkali-soluble photosensitive polymers used in the conventional photoresist composition can be used without limitation.

As a specific example, the base polymer may include a repeating unit represented by the following Chemical Formula a or Chemical Formula b. The base polymer may be a homopolymer consisting of one type of repeating unit represented by the Chemical Formula a, a homopolymer consisting of one type of repeating unit represented by the Chemical Formula b, a copolymer consisting of two or more types of repeating units represented by the Chemical Formula a, a copolymer consisting of two or more types of repeating units represented by the Chemical Formula b, or a copolymer consisting of one or more types of repeating units represented by the Chemical Formula a and one or more types of repeating units represented by the Chemical Formula b.

[Chemical Formula a]

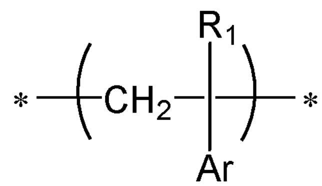

wherein, in Chemical Formula a, $R_1$ may be hydrogen or a methyl group, and Ar may be an aryl group in which at least one hydrogen atom is substituted or unsubstituted. The aryl group may be monocyclic or polycyclic. Examples of the substituents are not particularly limited, and may include, for example, one or more substituents selected from the group consisting of deuterium; a halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyloxy group; an aryloxy group; an alkylthioxy group; an arylthioxy group; an alkyl sulphoxy group; an aryl sulfoxy group; a silyl group; and a boron group. More specifically, a halogen or a hydroxy group may be used as the substituent.

[Chemical Formula b]

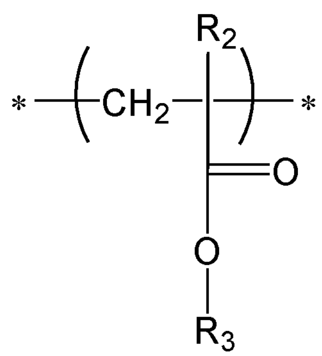

wherein, in Chemical Formula b, $R_2$ may hydrogen or a methyl group, and $R_3$ may be one of an alkyl group, an alkenyl group, a cycloalkyl group, a cycloalkenyl group, or an aryl group. At least one hydrogen atom included in the alkyl group, the alkenyl group, the cycloalkyl group, the cycloalkenyl group and the aryl group may be each independently unsubstituted or substituted with one or more substituents selected from the group consisting of deuterium; a halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyloxy group; an aryloxy group; an alkylthioxy group; an arylthioxy group; an alkyl sulphoxy group; an aryl sulfoxy group; a silyl group; and a boron group.

At least one carbon atom included in the alkyl group, the alkenyl group, the cycloalkyl group, the cycloalkenyl group, and the aryl group may be each independently substituted or unsubstituted with a hetero element.

The alkyl group and the alkenyl group are each independently straight chain or branched chain, and the cycloalkyl group, the cycloalkenyl group, and the aryl group may be each independently monocyclic or polycyclic.

More specifically, the base polymer may be a photosensitive polymer (base resin) represented by the following Chemical Formula c.

[Chemical Formula c]

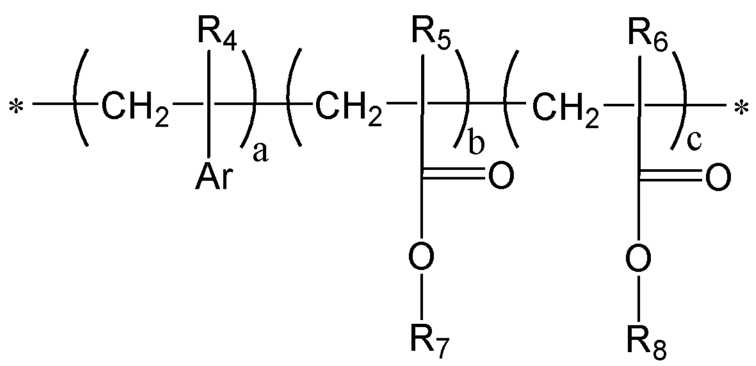

wherein, in Chemical Formula c, $R_4$ to $R_6$ are the same as or different from each other and are each independently hydrogen or a methyl group, Ar is an aryl group in which at least one hydrogen atom is substituted or unsubstituted, $R_7$ and $R_8$ are the same as or different from each other, and may be each independently one of an alkyl group, an alkenyl group, a cycloalkyl group, a cycloalkenyl group, or an aryl group. The Ar is the same as Ar in Chemical Formula a, and the $R_7$ and $R_8$ are the same as $R_3$ in Chemical Formula b.

More specifically, in Chemical Formula c, Ar may be a monocyclic aryl group having 6 to 11 carbon atoms in which at least one hydrogen atom is substituted with a hydroxy group.

In Chemical Formula c, the $R_7$ may be a monocyclic or polycyclic cycloalkyl group in which at least one carbon atom is substituted with a hetero element, or a polycyclic cycloalkyl group in which at least one hydrogen atom is substituted with a hydroxy group.

In Chemical Formula c, the $R_8$ may be a monocyclic or polycyclic cycloalkyl group.

In Chemical Formula c, a, b and c described below the repeating unit are mol% of each repeating unit relative to the total monomers (repeating units) forming the polymer, wherein a may be 10 mol% or more and 60 mol% or less, or 35 mol% or more and 40 mol% or less, b may be 1 mol% or more and 30 mol% or less, or 5 mol% or more and 20 mol% or less, and c may be 30 mol% or more and 60 mol% or less, or 40 mol% or more and 55 mol% or less. If the mol% of the repeating unit is out of the above range, there is a risk that the physical properties of the photoresist film may deteriorate, the formation of the photoresist film may become difficult, and the contrast of the pattern may deteriorate. Typically, the weight average molecular weight(Mw) of the photosensitive polymer may be 2,000 or more and 20,000 or less, or 3,000 or more and 12,000 or less.

More specific examples of the photosensitive polymer (base resin) represented by Chemical Formula c include a photosensitive polymer (base resin) represented by the following Chemical Formulas d and e, but are not limited thereto.

[Chemical Formula d]

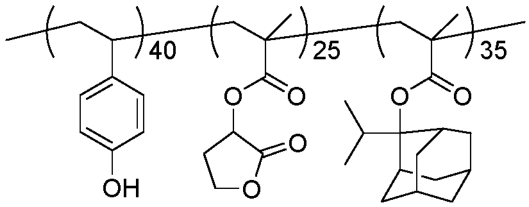

[Chemical Formula e]

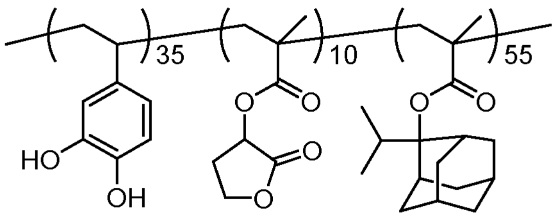

The content of the base polymer may be 2% by weight or more and 30% by weight or less, or 4% by weight or more and 10% by weight or less with respect to the total photoresist composition. If the content of the base polymer is less than 2% by weight, it may be difficult to form a photoresist film and pattern, and if the content is more than 30% by weight, the thickness distribution of the pattern formed on the wafer may become non-uniform.

Meanwhile, the photoresist composition according to one embodiment of the disclosure may or may not further contain an acid diffusion control agent (i.e., quencher) to improve resist performance. Specifically, the photoresist composition may include an acid diffusion inhibitor.

Examples of the acid diffusion inhibitor include conventional basic compounds such as primary, secondary, and tertiary aliphatic or aromatic amines, alcoholic nitrogen-containing compounds, amides, imides, and carbamates. By adding such a basic compound as an acid diffusion inhibitor, the acid diffusion rate within the resist thin film can be adjusted, thereby correcting and adjusting the pattern shape.

Further, instead of the alcoholic nitrogen-containing organic compound, photodegradable acid diffusion inhibitors that have a structure similar to that of photoacid generators and are decomposed by the exposure light source may be used. Such photodegradable acid diffusion inhibitors function as a quencher that suppresses acid diffusion in the non-exposed portion, and photolyze and function as acids in the exposed portion, thereby keeping the acid concentration in the exposed portion relatively high, increasing the acid distribution contrast between the exposed portion and the non-exposed portion, and improving lithographic characteristics. Onium salts such as sulfonium salts and iodonium salts of such as carboxylic acids, non-fluorinated sulfonic acids, or N-sulfonylimic acids may be used as the photodegradable acid diffusion inhibitor, and they may be used alone or in combination of two or more. In addition to the above-mentioned quencher, base polymer resin, and acid generator, other components such as an organic solvent, a surfactant, and a dissolution inhibitor can be used in appropriate combination to form a resist material.

The content of the acid diffusion inhibitor may be 1 part by weight or more and 90 parts by weight or less, preferably 5 parts by weight or more and 80 parts by weight or less, more preferably 10 parts by weight or more and 70 parts by weight or less, with respect to 100 parts by weight of the base polymer. If the content of the photoacid diffusion inhibitor is 1 part by weight or less, the sensitivity to light increases, but the content is too small, making it difficult to adjust the diffusion distance of the generated acid, and the pattern contrast of the exposed/non-exposed portion decreases. If the content of the photodiffusion inhibitor is more than 90 parts by weight, the sensitivity to light is weakened, and the exposure sensitivity may slow down or a reduction in resolving power may occur. In addition, in terms of optimizing the increase of sensitivity to light, contrast of exposed and unexposed portions, adjustment of diffusion distance, and improvement in resolving power, the content of the acid diffusion inhibitor may be 5 parts by weight or more and 80 parts by weight or less, or 10 parts by weight or more and 70 parts by weight or less.

As the organic solvent used in the present disclosure, any organic solvent used in conventional photoresist compositions that can easily dissolve the base polymer, the photoacid generator, the acid diffusion inhibitor, other additives, and the like can be used without limitation. For example, as the organic solvent, ketones such as cyclohexanone and methyl amyl ketone, alcohols such as 2-methoxybutanol, 3-methyl-3-methoxybutanol, 1-methoxy-2-propanol, and 1-ethoxy-2-propanol, ethers such as propylene glycol monomethyl ether, ethylene glycol monomethyl ether, propylene glycol monoethyl ether, ethylene glycol monoethyl ether, and diethyl glycol dimethyl ether, esters such as propyleneglycol

monomethyl etheracetate, propyleneglycol monoethyl etheracetate, ethyllactate, ethyl pyruvate, butyl acetate, methyl 3-methoxypropionate, 3-ethoxypropionate, tert-butylacetate, tert-butyl propionate, propylene glycol mono- tert-butyl ether acetate, lactones such as γ-butyrolactone can be used alone or in combination of two or more, but are not limited thereto. Examples of the organic solvent are not particularly limited, but as an example, any one of PGMEA (propylene glycol monomethyl ether acetate), PGME (propylene glycol monomethyl ether), EL (ethyl lactate), CyH (cyclohexanone), or a mixture thereof, which have the best solubility in an acid diffusion inhibitor, may be used.

The content of the organic solvent is the remainder obtained by excluding the content of the photoresist composition excluding the base polymer, the photoacid generator, the salt compound, and other additives, with respect to 100% by weight of the total photoresist composition.

Examples of the additives are not particularly limited, but for example, an additional resin, a stabilizer, a surfactant, a thickener, a defoaming agent, an adhesion agent, an antioxidant, a dissolution inhibitor, and the like may be used.

## Method for forming photoresist pattern

According to yet another embodiment of the present disclosure, there is provided a method for forming a photoresist pattern comprising the steps of: coating and heating the photoresist composition to form a photoresist film; exposing the photoresist film; and developing the exposed photoresist film to form a photoresist pattern.

The details regarding the photoresist composition includes the contents described above regarding the other embodiments.

The pattern forming method may apply a known technique, and for example, includes coating the composition by a spinner, etc., exposing the photoresist film to high-energy rays with a wavelength of 300 nm or less using a predetermined photo mask, and developing the exposed photoresist film with a conventional developer (e.g., an alkaline aqueous solution such as an aqueous tetramethylammonium hydroxide solution of 0.1% by weight or more and 10% by weight or less).

Specifically, the pattern forming method may form a pattern through the steps of: coating the above-mentioned photoresist composition onto a silicon wafer substrate, heating the applied substrate to form a resist thin film, exposing a resist thin film using a selected high-energy exposure source, removing an acid labile group in a polymer resin by acid generated in the exposed portion, and developing the resist thin film having changed solubility using the above developer.

The high-energy rays with a wavelength of 300 nm or less are not particularly limited, but ultraviolet rays, far-ultraviolet rays, extreme ultraviolet rays (EUV), electron beams, X-rays, excimer lasers, γ-rays, or synchrotron radiation may be exemplified. In order to form fine patterns of 70 nm or less, it is preferable to use an exposure device equipped with a source of short-wavelength high-energy rays, such as an ArF excimer laser, a KrF excimer laser, or an EUV.

More specifically, the high-energy exposure source can be selected from among KrF with a 248nm wavelength, ArF with a 193nm wavelength, e-beam, and EUV with a 13.5nm wavelength.

## [Advantageous Effects]

The photoacid generator according to the present disclosure can be used as an effective resist material to provide a photoresist composition that has improved polarity and shortens the acid diffusion length, thereby ensuring high contrast, improving a marginal resolving power, having a low LER/LWR value, and simultaneously satisfying CDU improvement, and further having a wide process margin.

In addition, by increasing the electron beam or EUV photon absorption, it is possible to generate more secondary electrons in the resist thin film, thereby improving sensitivity, and maximizing the resist performance improvement by containing a halogen element, especially iodine, having a higher light absorption ability for EUV light sources.

## [BRIEF DESCRIPTION OF THE DRAWINGS]

FIG. 1 shows $^1$H NMR data of the photoacid generator 1 of Synthesis Example 1 according to a preferred embodiment.
FIG. 2 shows $^1$H NMR data of the photoacid generator 2 of Synthesis Example 2 according to a preferred embodiment.
FIG. 3 shows $^1$H NMR data of the photoacid generator 3 of Synthesis Example 3 according to a preferred embodiment.

## [DETAILED DESCRIPTION OF THE EMBODIMENTS]

Below, a preferred example is presented to help the understanding of the present disclosure. However, the following Examples are for illustrative purposes only and are not intended to limit the present disclosure.

**<Synthesis Examples 1 to 9: Preparation of photoacid generator>**

**Synthesis Example 1: Preparation of photoacid generator 1**

**Synthesis Example 1-1:** Synthesis of sodium 1,1-difluoro-2-methoxy-2-oxoethanesulfonate

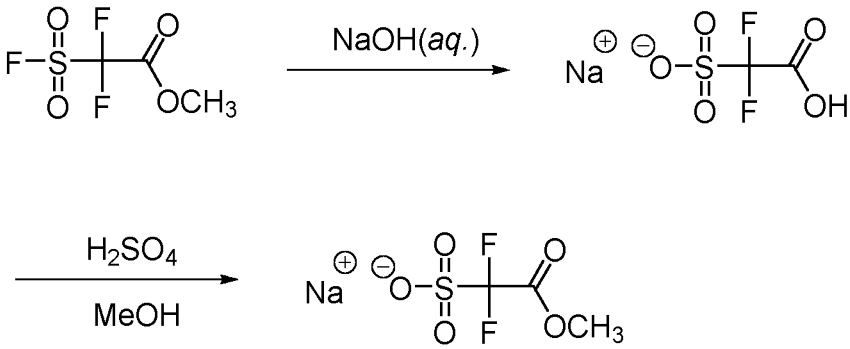


300 g of methyl 2-(fluorosulfonyl)difluoroacetate was slowly added dropwise to 5N sodium hydroxide aqueous solution in a 3L round bottom flask, and then stirred for 3 hours, and adjusted to the pH 3~5 with 10% with 10% hydrochloric acid solution, and the mixture was distilled under reduced pressure to remove water used as a solvent. Then, 1200 g of methanol was put in a flask containing the product distilled under reduced pressure, and the product was dissolved, and 25g of sulfuric acid was slowly added dropwise. After raising the temperature to 65□, the reaction mixture was stirred for 12 hours.

After the reaction was completed, insoluble inorganic substances (i.e. inorganic salts not participating in the reaction) was removed by filtration, concentrated under reduced pressure, and then 1L of a 2:1 (volume ratio) mixed solvent of methanol/ethanol was again added thereto. The insoluble inorganic salts were further removed by filtration, and concentrated under reduced pressure to obtain 285.7 g (yield: 95.29%) of 1,1-difluoro-2-methoxy-2-oxyethane sulfonate sodium salt without additional purification.
600MHz $^1$H-NMR (DMSO_d6, TMS): 3.78($CH_3$, s, 3H)

**Synthesis Example 1-2:** Synthesis of 1,1-difluoro-2-hydroxyethanesulfonate sodium salt

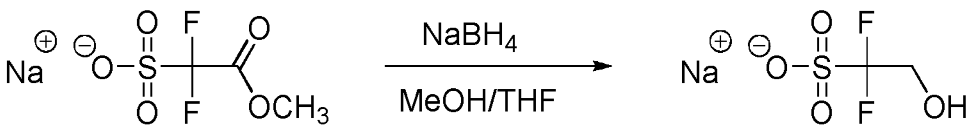


280 g of 1,1-difluoro-2-methoxy-2-oxyethane sulfonate sodium salt obtained through Synthesis Example 1-1 was put in a 3L round bottom flask (reactor), and 0.6L of methanol and 1.5L of tetrahydrofuran were added and dissolved, and the reactor was cooled to -10°C. Then, 175 g of sodium borohydride was slowly added to the reactor, the temperature was raised to room temperature, and then the mixture was stirred. The temperature was again raised to 60□, and the mixture was stirred for 24 hours.

After the reaction was completed, the reaction solution was acidified to pH 3~5 using concentrated hydrochloric acid, and the insoluble inorganic materials were removed by filtration and then the solvent was concentrated under reduced pressure. 1 L of a mixed solvent of methanol/ethanol in a 2:1 ratio (volume ratio) was again added thereto, insoluble inorganic salts were further removed by filtration, and the solvent was concentrated under reduced pressure to obtain 237.2 g (yield: 97.6%) of sodium 1,1-difluoro-2-hydroxyethane sulfonate salt without additional purification.
600MHz $^1$H-NMR ($D_2O$, TMS): 4.12($CH_2$, t, 2H)

**Synthesis Example 1-3:** Synthesis of 1,1-difluoro-2-hydroxyethanesulfonate triphenylsulfonium salt

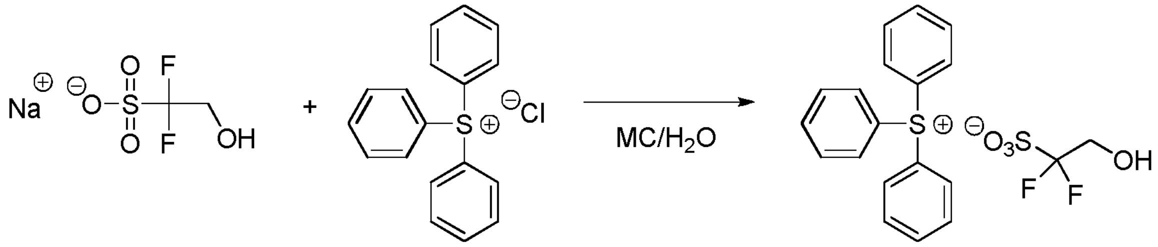


150 g of sodium 1,1-difluoro-2-hydroxyethane sulfonate salt obtained through Synthesis Example 1-2, and 268g of triphenylsulfonium chloride were put in a 2,000L round bottom flask, to which 1,000g of dichloromethane and 200g of distilled water were added and dissolved, and the mixture was then stirred at room temperature for 12 hours. The organic layer was separated and then distilled under reduced pressure.

The solid product produced through reduced pressure distillation was added to and dissolved in 800 g of ethanol, and the insoluble inorganic salt was removed by filtration, solidified using 5L of diethyl ether, further stirred for 1 hour, filtered and dried to obtain 321.6 g (yield: 92.98%) of 1,1-difluoro-2-hydroxyethanesulfonate triphenylsulfonium salt.
600MHz $^1$H-NMR ($CDCl_3$, TMS): 7.75(CH, m, 15H), 4.05($CH_2$, t, 2H), 3.6(OH)

**Synthesis Example 1-4:** Synthesis of 1,1-difluoro-2-(4-iodophenylsulfonyloxy)ethanesulfonate triphenylsulfonium salt

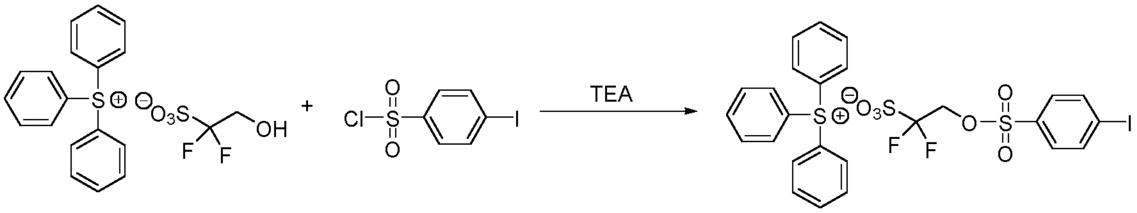


(PAG 1)

200 g of 1,1-difluoro-2-hydroxyethanesulfonate triphenylsulfonium salt obtained through Synthesis Example 1-3 was put in a 3L round bottom flask, to which 1,200g of dichloromethane was added and dissolved. 57 g of triethylamine was diluted in 300 g of dichloromethane and added to the flask. Next, 171 g of 4-iodophenyl sulfonyl chloride was dissolved in 1,200 g of dichloromethane solvent, and added slowly for 1 hour. The mixture was then stirred at room temperature for 5 hours. Next, the resulting sodium chloride was removed by filtration, and the organic layer was washed three times with 1,500 g of distilled water each. The organic layer was separated and then distilled under reduced pressure.

The resulting solid product was added to and dissolved in 600 g of methanol and then solidified using 5 L of diethyl. The mixture was further stirred for 1 hour, filtered and dried to obtain 296.8 g (yield: 91.22%) of 1,1-difluoro-2-(4-iodophenylsulfonyloxy)ethanesulfonate triphenylsulfonium salt.
600MHz $^1$H-NMR (DMSO_d6): 8.07(CH, d, 2H), 7.80(CH, m, 15H), 7.69(CH, d, 2H), 4.49($CH_2$, t, 2H)

**Synthesis Example 2: Preparation of photoacid generator 2**

Synthesis of 1,1-difluoro-2-(4-trifluoromethanophenylsulfonyloxy)ethanesulfonate triphenylsulfonium salt

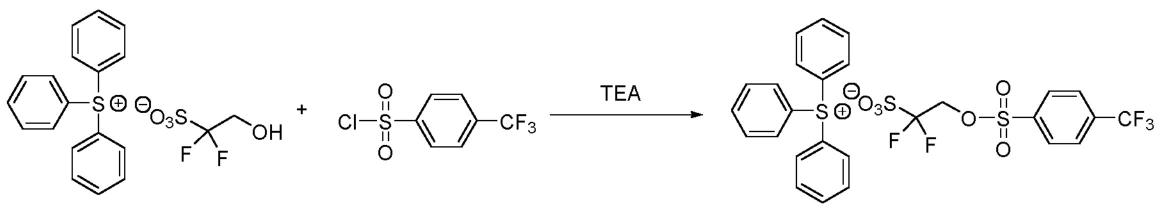


(PAG 2)

The remaining reactions were carried out in the same manner as in Synthesis Examples 1-1 to 1-4, except that in Synthesis Example 1-4, 138 g of 4-trifluoromethane phenyl sulfonyl chloride was used instead of 4-iodophenyl sulfonyl chloride. Thereby, 268.36 g (yield: 88.35%) of 1,1-difluoro-2-trifluoromethane phenylsulfonyloxyethane sulfonate triphenylsulfonium salt was obtained.
600MHz $^1$H-NMR $(CD_3)_2CO$, TMS): 8.25(CH, d, 2H), 8.15(CH, d, 2H), 7.90(CH, m, 15H), 4.64($CH_2$, t, 2H)

**Synthesis Example** 3: **Preparation of photoacid generator** 3

Synthesis of 1,1,2,2-tetrafluoro-3-iodophenylsulfonyloxyethanesulfonate triphenylsulfonium salt

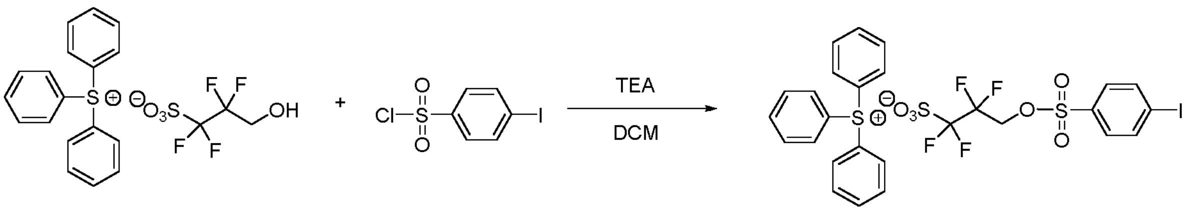


(PAG 3)

The remaining reactions were carried out in the same manner as in Synthesis Examples 1-1 to 1-4, except that in Synthesis Example 1-1, 150 g of methyl 2,2,3,3-tetrafluoro-3-(fluorosulfonyl)propanoate was used instead of methyl 2-(fluorosulfonyl)difluoroacetate as the starting material. Thereby, 213.45 g (yield: 91.2%) of 1,1,2,2-tetrafluoro-3-iodophenyloxyethanesulfonate triphenylsulfonium salt was obtained.
600MHz $^1$H-NMR $(CD_3)_2CO$, TMS): 8.12(CH, d, 2H), 7.90(CH, m, 15H), 7.49(CH, d, 2H), 4.60($CH_2$, t, 2H)

**Synthesis Examples 4 to 16: Preparation of photoacid generators 4 to 16**

Anionic compounds having the following sulfonyloxy group and carbonyloxy group substituents were prepared by applying the methods of Synthesis Examples 1 to 3 and changing the type of the starting material of Synthesis Example 1-1, the cationic compound of Synthesis Example 1-3, and the anionic -R-EWG-providing compound of Synthesis Example 1-4. Subsequently, through an ion exchange reaction between the anionic compound and sulfonium chloride having the following cation, photoacid generators 4 to 16 having the following structures were prepared, respectively.

**Photoacid generator 4:**

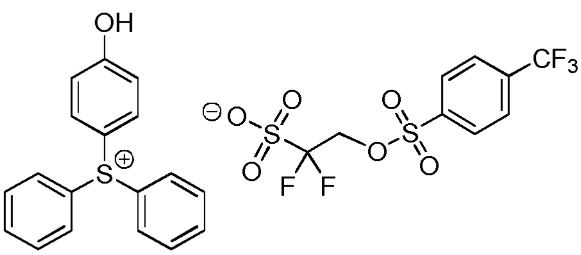

Yield: 72.52%,
600MHz $^1$H-NMR ($CDCl_3$, TMS): 8.02(CH, d, 2H), 7.91(CH, d, 2H), 7.52(CH, m, 10H), 7.33(CH, 2H), 7.11(CH, 2H), 5.56(OH), 4.43($CH_2$, t, 2H)

**Photoacid generator 5:**

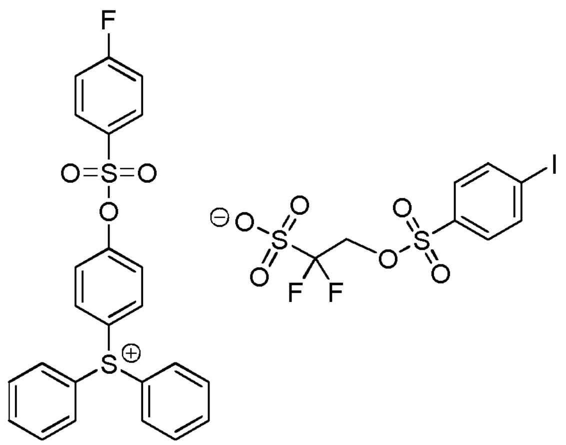

Yield: 78.35%,
600MHz $^{1}$H-NMR ($CDCl_3$, TMS): 8.24(CH, d, 2H), 7.92(CH, 2H), 7.70(CH, d, 2H), 7.40~7.48(CH, m, 12H), 6.85~7.30(CH, m, 4H), 7.11(CH, 2H), 4.44($CH_2$, t, 2H)

**Photoacid generator 6:**

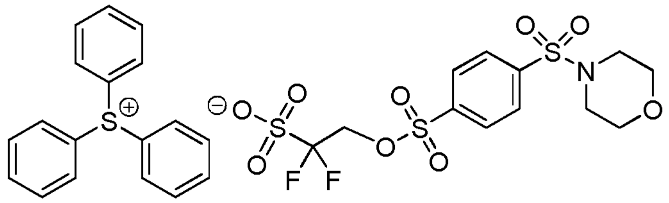

Yield: 75.10%,
600MHz $^{1}$H-NMR ($CDCl_3$, TMS): 8.11~8.22(CH, m, 4H), 7.48(CH, m, 15H), 4.56($CH_2$, t, 2H), 3.70($CH_2$, m, 4H), 3.02($CH_2$, m, 4H)

**Photoacid generator 7:**

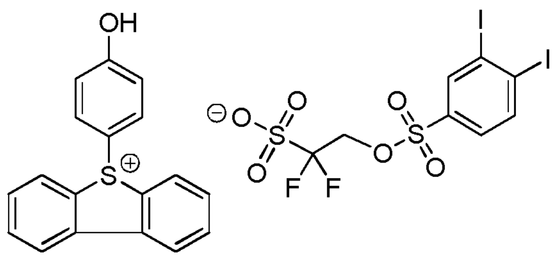

Yield: 87.50%,
600MHz $^{1}$H-NMR ($CDCl_3$, TMS): 8.12(CH, d, 1H), 7.92(CH, d 1H), 7.75(CH, t, 1H), 7.40~7.80(CH, m, 8H), 6.98~7.32(CH, m, 4H), 5.40(OH), 4.48($CH_2$, t, 2H)

**Photoacid generator 8:**

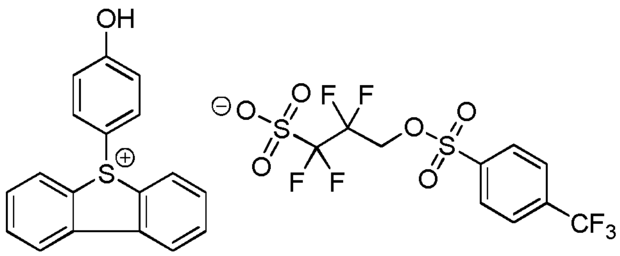

Yield: 78.38%,
600MHz $^1$H-NMR ($CDCl_3$, TMS): 8.02(CH, d, 2H), 7.84(CH, d, 2H), 7.40~7.80(CH, m, 8H), 6.96~7.30(CH, m, 4H), 5.20(OH), 4.02($CH_2$, t, 2H)

**Photoacid generator 9:**

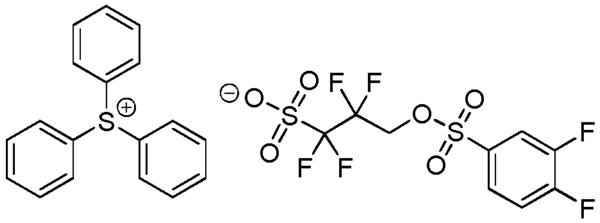

Yield: 80.85%,
600MHz $^1$H-NMR ($CDCl_3$, TMS): 7.92(CH, d, 1H), 7.45~7.60(CH, m, 17H), 3.94($CH_2$, t, 2H)

**Photoacid generator 10:**

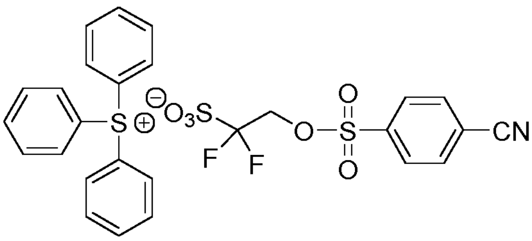

Yield: 85.27%,
600MHz $^1$H-NMR ($CDCl_3$, TMS): 7.96~8.10(CH, m, 4H), 7.49(CH, m, 15H), 4.52($CH_2$, t, 2H)

**Photoacid generator 11:**

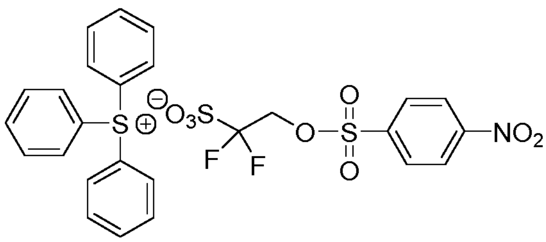

Yield: 86.62%,
600MHz $^1$H-NMR ($CDCl_3$, TMS): 8.44(CH, d, 2H), 8.10(CH, d, 2H), 7.50(CH, m, 15H), 4.56($CH_2$, t, 2H)

**Photoacid generator 12:**

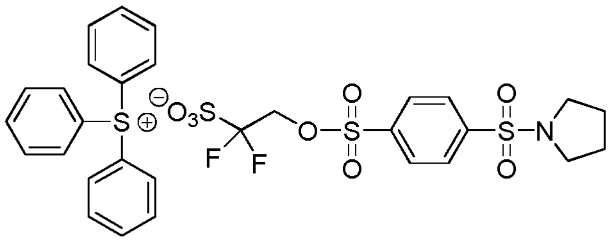

Yield: 83.18%,
600MHz $^1$H-NMR ($CDCl_3$, TMS): 8.15~8.30(CH, m, 4H), 7.48(CH, m, 15H), 4.48($CH_2$, t, 2H), 3.30($CH_2$, t, 4H), 1.94($CH_2$, t, 4H)

**Photoacid generator 13:**

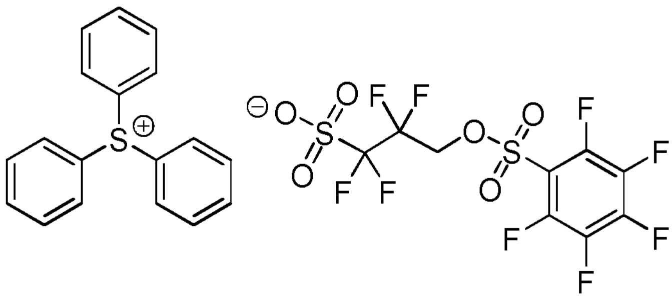

Yield: 77.92%,
600MHz $^1$H-NMR ($CDCl_3$, TMS): 7.48(CH, m, 15H), 4.02($CH_2$, t, 2H)

**Photoacid generator 14:**

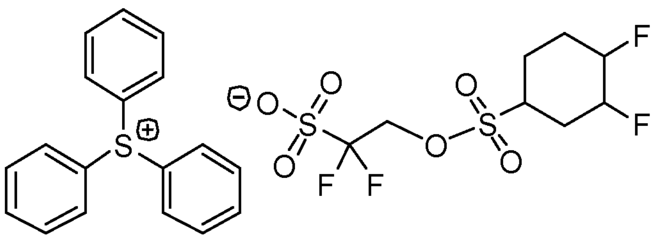

Yield: 72.16%,
600MHz $^1$H-NMR ($CDCl_3$, TMS): 7.48(CH, m, 15H), 4.48($CH_2$, t, 2H), 3.40~3.60(CH, m, 2H), 2.96(CH, m, 1H), 1.40~2.40($CH_2$, m, 6H)

**Photoacid generator 15:**

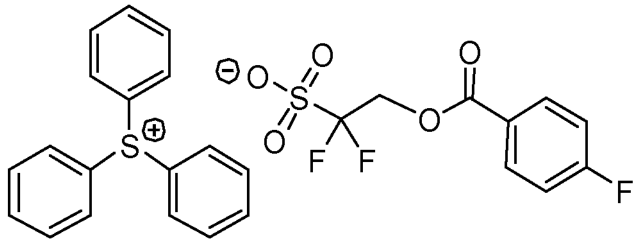

Yield: 83.22%,
600MHz $^1$H-NMR ($CDCl_3$, TMS): 8.04(CH, d, 2H), 7.38~7.48(CH, m, 17H), 5.04($CH_2$, t, 2H)

**Photoacid generator 16:**

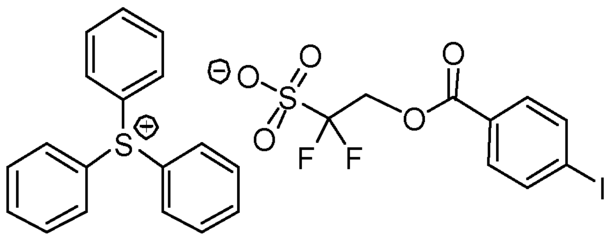

Yield: 85.15%,
600MHz $^1$H-NMR ($CDCl_3$, TMS): 7.84~7.92(CH, m, 4H), 7.48(CH, m, 15H), 5.04($CH_2$, t, 2H)

The structures of the photoacid generators according to Synthesis Examples 1 to 16 are as shown in Table 1 below.

[Table 1]

| | Structure |
|---|---|
| Photoacid generator 1 | 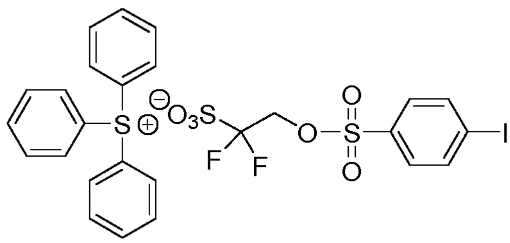 |
| Photoacid generator 2 | 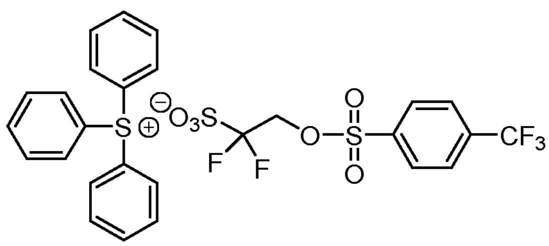 |

(continued)

| | Structure |
|---|---|
| Photoacid generator 3 | 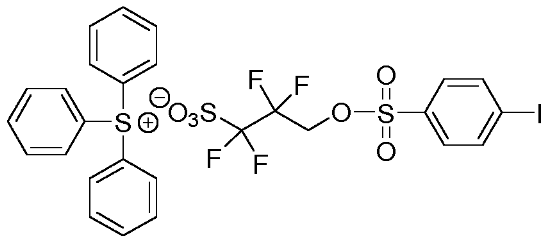 |
| Photoacid generator 4 | 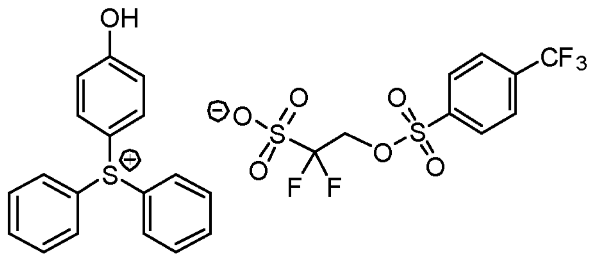 |
| Photoacid generator 5 | 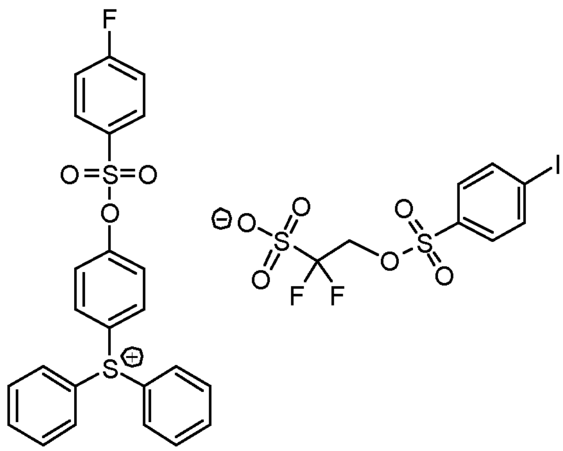 |
| Photoacid generator 6 | 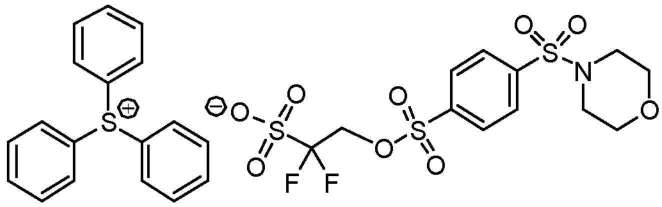 |
| Photoacid generator 7 | 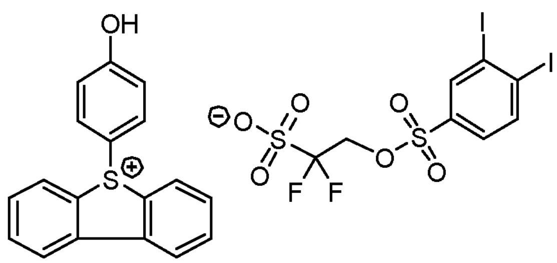 |

(continued)

| | Structure |
|---|---|
| Photoacid generator 8 | 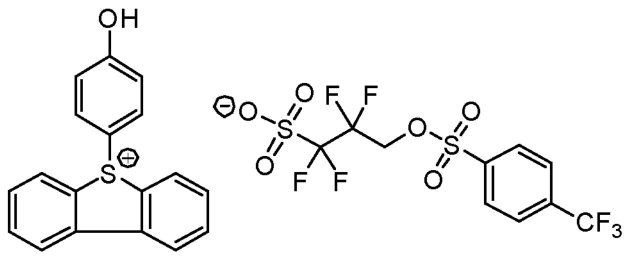 |
| Photoacid generator 9 | 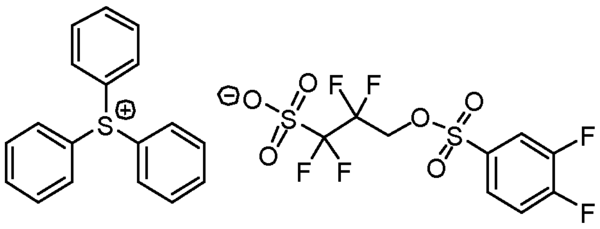 |
| Photoacid generator 10 | 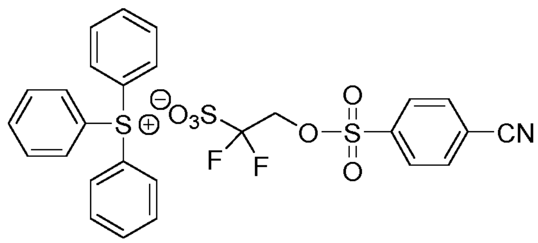 |
| Photoacid generator 11 | 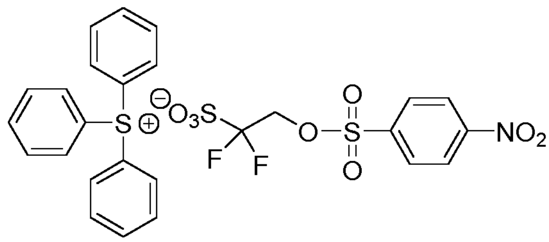 |
| Photoacid generator 12 | 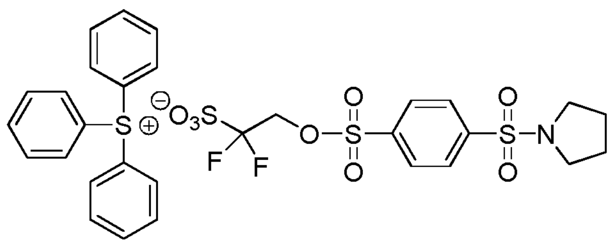 |
| Photoacid generator 13 | 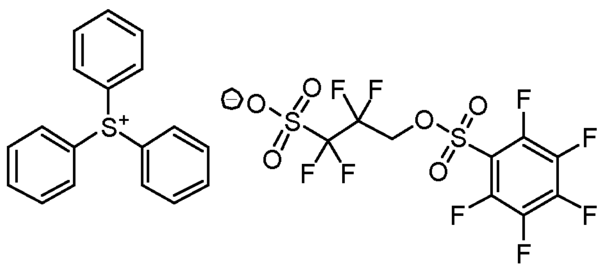 |

(continued)

| | Structure |
|---|---|
| Photoacid generator 14 | 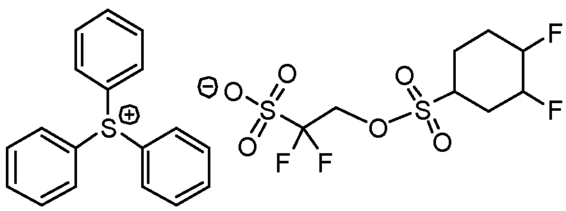 |
| Photoacid generator 15 | 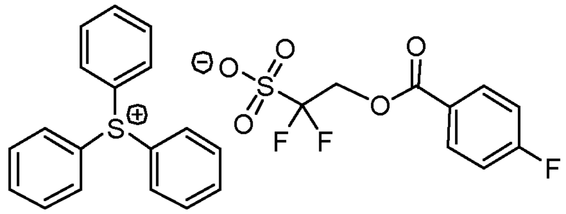 |
| Photoacid generator 16 | 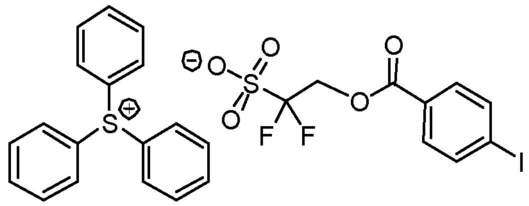 |

**<Synthesis Examples 2 and 3: Synthesis of base polymers (polymers 1 - 2)>**

Monomers were combined to have the repeating units shown in Table 2 below, and subjected to a copolymerization reaction in a tetrahydrofuran (THF) solvent. Then, for low molecule removal and solidification, the reaction solution was added dropwise to methanol, n-hexane, or hexane/isopropyl alcohol, or methanol/water mixed solvent, immersed, then washed repeatedly with the same solvent, separated and dried to prepare base polymers 1 and 2 containing a hydroxystyrene group in the aromatic ring.

The hydroxystyrene group-containing group can be obtained by carrying out a secondary hydrolysis reaction after a primary polymerization using a monomer protected with an acetoxy group, and then adding a precipitation process using water.

After drying the base polymers 1 and 2, the monomer components were analyzed by $^1$H or $^{13}$C-NMR spectroscopy, Mw and PDI (Mw/Mn) were analyzed using gel permeation chromatography (GPC), and the polymer molecular weight (Mw) expressed in terms of standard polystyrene was determined using THF solvent. The types and analysis results of the repeating units of polymers 1 and 2 are listed in Table 2 below.

[Table 2]

| Type | Structure, molecular weight, and PDI values of repeat units |
|---|---|
| Polymer 1 | 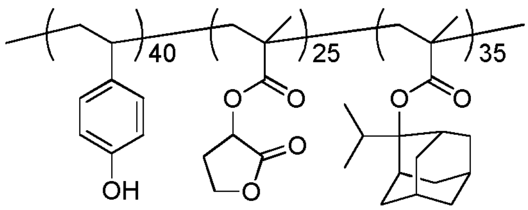 |
| | Mw=6,400, PDI(Mw/Mn)=1.44 |

(continued)

| Type | Structure, molecular weight, and PDI values of repeat units |
|---|---|
| Polymer 2 | 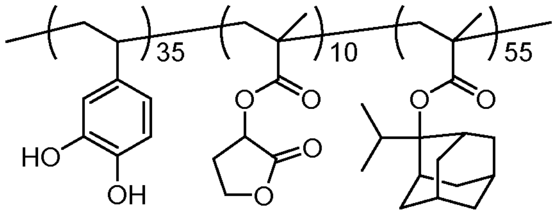 |
| | Mw=6,800, PDI(Mw/Mn)=1.49 |

**<Examples 1 to 25 and Comparative Examples 1 to 12>**

Preparation of photoresist composition

Based on the components listed in Table 3 below, 100 parts by weight of the base polymer and the selected components (25 parts by weight of photoacid generator and 15 parts by weight of quencher) were prepared, and these were dissolved in an organic solvent according to Table 3 below and mixed. Then, the mixture was filtered through a polytetrafluoroethylene (PTFE) filter with pores of 0.2$\mu$m to prepare photoresist compositions of Examples and Comparative Examples.

**«Base polymer»**

In Examples and Comparative Examples, one of the polymers 1 and 2 prepared in Synthesis Examples 2 and 3 was selected and used as a base polymer.

**<<Photoacid generator>>**

In Examples 1 to 25, one of the photoacid generators 1 to 16 was selected and used.
In Comparative Examples 1 to 12, one of photoacid generators A to E having the following structure was selected and used.

Photoacid generator A:

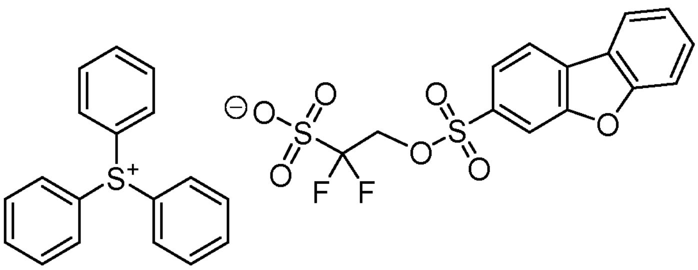

Photoacid generator B:

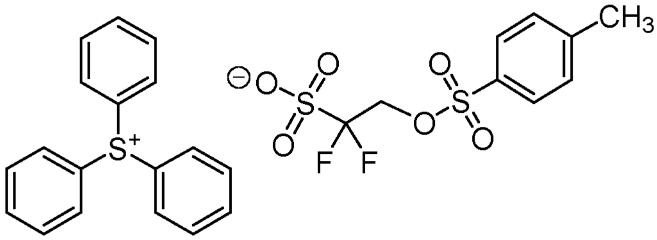

Photoacid generator C:

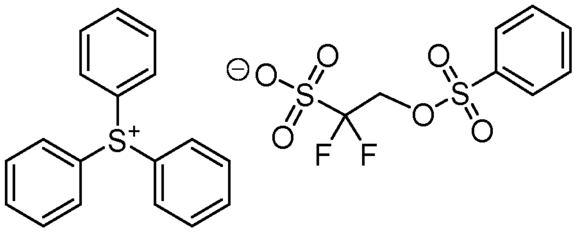

Photoacid generator D:

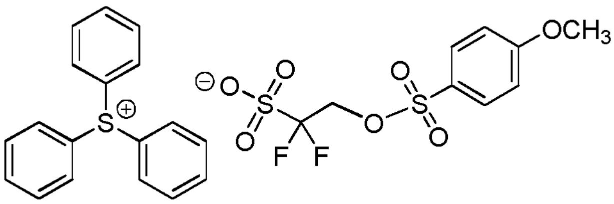

Photoacid generator E:

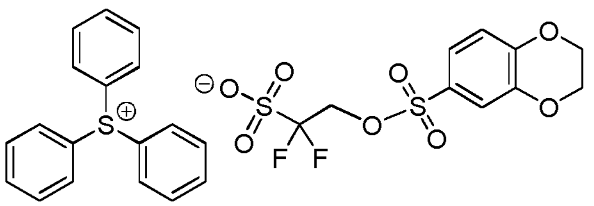

**<<Quencher; Salt Compound>>**

In Examples and Comparative Examples, one of Quencher A and Quencher B of the following structures was selected and used.

Quencher A:

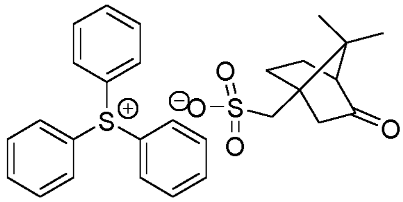

Quencher B:

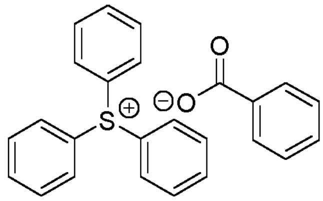

**<<Organic Solvent>>**

In Examples and Comparative Examples, one or more types of organic solvents selected from the following were used.

PGMEA (propylene glycol monomethyl ether acetate)
PGME (propylene glycol monomethyl ether)
EL (ethyl lactate)
CyH (cyclohexanone)

**[Experimental Example]**

**Formation of resist pattern**

To analyze resist pattern characteristics, an organic thin film under the resist was coated onto a silicon wafer to prepare a substrate, and the photoresist compositions of Examples and Comparative Examples were spin coated onto the substrate to form a resist thin film. Next, the resist thin film was heated and soft-baked (SOB) at a temperature of 100°C~130°C for 60 seconds using a hot plate, (i) exposed with ASML NXE:3400 EUV exposure equipment with a numerical aperture (N.A.) of 0.33, and then heated (post exposure bake, PEB) at a temperature of 100°C~130°C for 60 seconds. The thus heated wafer was developed with a 2.38 wt.% tetramethylammonium hydroxide (TMAH) aqueous solution for 30 seconds to form a 1:1 line and space (L/S: line/space) pattern with a film thickness of 35 nm and a line width of 18 nm. At this time, the SOB and PEB when forming the patterns of Examples and Comparative Examples showed 100°C.

To measure the pattern size on a wafer on which the pattern was formed by the above method, the pattern size was measured using a Critical Dimension Scanning Electron Microscope (CD-SEM, product name: CG-5000) from Hitachi High-Technologies Corp., and the sensitivity, line width roughness (LER), and process margin (EL: energy latitude, DOF: depth of focus) were evaluated using the following methods. The evaluation results are shown in Table 1 below.

**Evaluation of resist pattern characteristics**

**(1) Sensitivity evaluation (unit: mJ/cm$^2$)**

The size of the optimal exposure energy (Eop, unit: mJ/cm$^2$) at which a resist pattern having the same size space (pitch 18 nm) was formed for a resist line width of 18 nm was measured. As the sensitivity is lower, it can be evaluated as superior.

**(2) LER/LWR evaluation (Line Edge Roughness/Line Width Roughness; unit: nm)**

In the 18nm pattern formed at the optimal exposure energy(Eop), the roughness size (nm), which was calculated as the average value of three times(3s) the standard deviation(s) for the value of line edge roughness measured at 200 measurement points in the length direction of line width pattern, was evaluated. As the LER value is lower, it can be evaluated as superior.

**(3) EL margin evaluation (Exposure Latitude, unit: %)**

The EL margin (process margin) was calculated according to the following Equation using the exposure energy value when a 18 nm sized pattern formed at the optimal exposure energy (Eop) was formed within the range of 18 nm ± 5% line width (17.1nm ~ 18.9nm). As the EL margin value is higher, it can be evaluated as superior.

[Equation 1]

$$\text{EL margin (\%)} = (|E_1 - E_2|/Eop) \times 100$$

in Equation 1,

$E_1$: exposure energy value (mJ/cm$^2$) when forming a pattern with a line width of 17.1nm
$E_2$: exposure energy value (mJ/cm$^2$) when forming a pattern with a line width of 18.9 nm

**(4) DOF margin evaluation (Depth of focus, unit: nm)**

The DOF margin was calculated by a depth of focus change value having a value within the range of ±1.5% (17.73nm ~ 18.27nm) of the pattern line width of 18nm according to the change(±) in depth of focus (DOF) from the optimal exposure energy(Eop). That is, the DOF margin evaluation for this was calculated according to the following Equation 2. As the DOF margin value is higher, it can be evaluated as superior.

[Equation 2]

$$\text{DOF margin (nm)} = D_1 + D_2$$

in Equation 2,

$D_1$ is +depth of focus value (nm) when a pattern within ±1.5% of the line width is formed.
$D_2$ is -depth of focus value (nm) when a pattern within ±1.5% of the line width is formed

[Table 3]

| Example | | | | | | | | |
|---|---|---|---|---|---|---|---|---|
| No. | Base polyme r | Photoaci d generate r | Quench er | Organic solvent (part by weight) | Sensitivit y (mJ/cm$^2$) | LW R (nm ) | EL (%) | DOF (nm) |
| 1 | Polyme r 1 | Photoaci d generate r1 | Quench er A | PGMEA(80 0) EL (1200) | 56 | 3.58 | 10. 8 | 95 |
| 2 | Polyme r 1 | Photoaci d generate r2 | Quench er A | PGMEA(80 0) EL (1200) | 57 | 3.67 | 10. 3 | 90 |
| 3 | Polyme r 1 | Photoaci d generate r3 | Quench er A | PGMEA(80 0) EL (1200) | 55 | 3.55 | 10. 9 | 95 |
| 4 | Polyme r 1 | Photoaci d generate r4 | Quench er A | PGMEA(80 0) EL (1200) | 54 | 3.58 | 11. 2 | 95 |

(continued)

| Example | | | | | | | | |
|---|---|---|---|---|---|---|---|---|
| No. | Base polyme r | Photoaci d generate r | Quench er | Organic solvent (part by weight) | Sensitivit y (mJ/cm$^2$) | LW R (nm ) | EL (%) | DOF (nm) |
| 5 | Polyme r 1 | Photoaci d generate r5 | Quench er A | PGMEA(80 0) EL (1200) | 53 | 3.61 | 11. 5 | 100 |
| 6 | Polyme r 1 | Photoaci d generate r 6 | Quench er A | PGMEA(80 0) EL (1200) | 56 | 3.71 | 10. 4 | 100 |
| 7 | Polyme r 1 | Photoaci d generate r 7 | Quench er A | PGMEA(80 0) EL (1200) | 52 | 3.46 | 10. 8 | 100 |
| 8 | Polyme r 1 | Photoaci d generate r 8 | Quench er A | PGMEA(80 0) EL (1200) | 54 | 3.59 | 10. 2 | 95 |
| 9 | Polyme r 1 | Photoaci d generate r 9 | Quench er A | PGMEA(80 0) EL (1200) | 56 | 3.61 | 10. 7 | 95 |
| 10 | Polyme r 1 | Photoaci d generate r 10 | Quench er A | PGMEA(80 0) EL (1200) | 55 | 3.54 | 9.8 | 100 |
| 11 | Polyme r 1 | Photoaci d generate r 11 | Quench er A | PGMEA(80 0) EL (1200) | 54 | 3.64 | 10. 4 | 95 |
| 12 | Polyme r 1 | Photoaci d generate r 12 | Quench er A | PGMEA(80 0) EL (1200) | 57 | 3.49 | 10. 0 | 95 |
| 13 | Polyme r 1 | Photoaci d generate r 13 | Quench er A | PGMEA(80 0) EL (1200) | 53 | 3.66 | 9.8 | 95 |
| 14 | Polyme r 1 | Photoaci d generate r 14 | Quench er A | PGMEA(80 0) EL (1200) | 66 | 3.71 | 10. 5 | 95 |
| 15 | Polyme r 1 | Photoaci d generate r 15 | Quench er A | PGMEA(80 0) EL (1200) | 65 | 3.75 | 10. 2 | 95 |
| 16 | Polyme r 1 | Photoaci d generate r 16 | Quench er A | PGMEA(80 0) EL (1200) | 63 | 3.69 | 10. 4 | 100 |
| 17 | Polyme r 2 | Photoaci d generate r 1 | Quench er B | PGMEA(80 0) PGME(120 0) | 51 | 3.34 | 11. 8 | 100 |
| 18 | Polyme r 2 | Photoaci d generate r 2 | Quench er B | PGMEA(80 0) PGME(120 0) | 52 | 3.26 | 10. 7 | 100 |
| 19 | Polyme r 2 | Photoaci d generate r 3 | Quench er B | PGMEA(80 0) PGME(120 0) | 51 | 3.30 | 10. 8 | 105 |
| 20 | Polyme r 2 | Photoaci d generate r 4 | Quench er B | PGMEA(80 0) PGME(120 0) | 51 | 3.25 | 10. 5 | 100 |
| 21 | Polyme r 2 | Photoaci d generate r 5 | Quench er B | PGMEA(80 0) PGME(120 0) | 52 | 3.38 | 11. 4 | 105 |
| 22 | Polyme r 2 | Photoaci d generate r 6 | Quench er B | PGMEA(80 0) PGME(120 0) | 53 | 3.47 | 11 | 110 |
| 23 | Polyme r 2 | Photoaci d generate r 7 | Quench er B | PGMEA(80 0) CyH (1200) | 51 | 3.32 | 10. 7 | 105 |
| 24 | Polyme r 2 | Photoaci d generate r 8 | Quench er B | PGMEA(80 0) CyH (1200) | 53 | 3.36 | 10. 4 | 100 |

| | | | | | | | | |
|---|---|---|---|---|---|---|---|---|
| 25 | Polyme r 2 | Photoaci d generate r 9 | Quench er B | PGMEA(80 0) PGME(120 0) | 54 | 3.31 | 10. 2 | 105 |

(continued)

| Comparative Example | | | | | | | | |
|---|---|---|---|---|---|---|---|---|
| 1 | Polyme r 1 | Photoaci d generate r A | Quench er A | PGMEA(80 0) EL (1200) | 64 | 4.31 | 8.9 | 80 |
| 2 | Polyme r 1 | Photoaci d generate r A | Quench er B | PGMEA(80 0) PGME(120 0) | 67 | 3.97 | 9.2 | 90 |
| 3 | Polyme r 2 | Photoaci d generate r A | Quench er A | PGMEA(80 0) EL (1200) | 62 | 4.28 | 9.7 | 80 |
| 4 | Polyme r 2 | Photoaci d generate r A | Quench er B | PGMEA(80 0) PGME(120 0) | 65 | 3.93 | 9.8 | 95 |
| 5 | Polyme r 1 | Photoaci d generate r B | Quench er A | PGMEA(80 0) EL (1200) | 63 | 4.11 | 9.2 | 85 |
| 6 | Polyme r 1 | Photoaci d generate r B | Quench er B | PGMEA(80 0) PGME(120 0) | 65 | 3.91 | 9.7 | 95 |
| 7 | Polyme r 2 | Photoaci d generate r B | Quench er A | PGMEA(80 0) EL (1200) | 64 | 3.96 | 8.8 | 95 |
| 8 | Polyme r 2 | Photoaci d generate r B | Quench er B | PGMEA(80 0) PGME(120 0) | 66 | 3.89 | 9.5 | 95 |
| 9 | Polyme r 1 | Photoaci d generate r C | Quench er A | PGMEA(80 0) PGME(120 0) | 62 | 3.85 | 9.2 | 90 |
| 10 | Polyme r 1 | Photoaci d generate r C | Quench er B | PGMEA(80 0) PGME(120 0) | 63 | 3.80 | 9.5 | 95 |
| 11 | Polyme r 2 | Photoaci d generate r D | Quench er B | PGMEA(80 0) PGME(120 0) | 65 | 3.82 | 9.7 | 90 |
| 12 | Polyme r 1 | Photoaci d generate r E | Quench er B | PGMEA(80 0) PGME(120 0) | 68 | 3.84 | 9.5 | 95 |

Looking at the results in Table 3, it was confirmed that Examples 1 to 25 exhibited lower sensitivity and LWR and higher EL and DOF process margins compared to Comparative Examples 1 to 12.

That is, when the photoresist compositions of Examples 1 to 25 were used, a compound containing R-EWG in which in Chemical Formula 1, Y contains an alkylene group having 2 to 5 carbon atoms substituted with 2 to 4 F, A contains a sulfonate group, Z contains a sulfonyloxy group or carbonyloxy group, and the end has a specific structure, was used as a photoacid generator, thereby exhibiting a sensitivity of 51 $mJ/cm^2$ or more and 66 $mJ/cm^2$ or less, LWR of 3.25 nm or more and 3.71 nm or less, EL margin of 10.2% or more and 11.8% or less, and excellent DOF margin of 90 nm or more and 110 nm or less. At this time, Examples 1 to 13 and Examples 17 to 25 exhibited a relatively shorter acid diffusion distance and a better effect than Examples 15 and 16 in which in Chemical Formula 1, Z contains a sulfonyloxy group, A is a sulfonate group, and Z is a carbonyloxy group. In addition, Examples 14 to 16 exhibited similar sensitivities as the Comparative Examples, but had lower LWR and EL and more excellent DOF process margin than Comparative Examples, thereby improving resist performance more than Comparative Examples.

On the other hand, when the photoresist compositions of Comparative Examples 1 to 12 were used, the sensitivity was wholly higher than Examples whose sensitivity was 62 $mJ/cm^2$ or more and 68 $mJ/cm^2$ or less. In particular, Comparative Examples 1 to 12 exhibited an LWR of 3.80 nm or more and 4.31 nm or less, an EL margin of 8.8% or more and 9.8% or less, and a DOF margin of 80 nm or more and 95 nm or less, confirming that they had poorer physical properties than Examples.

From these results, the Examples have lower sensitivity and LWR and can improve both EL and DOF process margins as compared to Comparative Examples, and thus, can be suitably used as chemically amplified resist compositions having high resolution, which can contribute to the performance improvement of highly integrated semiconductor devices.

**Claims**

1. A photoacid generator comprising an anion represented by the following Chemical Formula 1, and a cation represented by the following Chemical Formula 2:

[Chemical Formula 1] A-Y-Z-R-EWG

[Chemical Formula 2]

$$M^{\oplus}$$

wherein, in Chemical Formulas 1 and 2,
A and Z are the same as or different from each other, and are each independently a polar functional group,
Y is a linker connecting A and Z, which is a divalent organic group,
R represents a chain or cyclic aliphatic hydrocarbon group or a monocyclic or polycyclic aromatic hydrocarbon group,
EWG is an electron withdrawing group substituted for R, and
$M^+$ is an onium ion or a metal ion.

2. The photoacid generator according to claim 1, wherein:
the A is either a sulfonate group or a carbonate group.

3. The photoacid generator according to claim 1, wherein:
the A is a sulfonate group.

4. The photoacid generator according to claim 1, wherein:
the Y is any one of an alkylene groups having 1 to 10 carbon atoms substituted with an atomic group containing at least one F.

5. The photoacid generator according to claim 1, wherein:
the Y is any one of an alkylene groups having 2 to 5 carbon atoms substituted with 2 to 4 F.

6. The photoacid generator according to claim 1, wherein:
the Z is either a sulfonyloxy group (-SOs-) or a carbonyloxy group (-C(=O)O-).

7. The photoacid generator according to claim 1, wherein:
the Z is either a sulfonyloxy group (-SOs-).

8. The photoacid generator according to claim 1, wherein:

the chain or cyclic aliphatic hydrocarbon group is an alkyl group, an alkenyl group, a cycloalkyl group, or a cycloalkenyl group, which is substituted with one or more electron withdrawing groups (EWG),
the alkyl group and the alkenyl group are each independently a straight chain or branched chain group having 1 to 30 carbon atoms, and
the cycloalkyl group or the cycloalkenyl group is each independently a monocyclic or polycyclic group having 3 to 30 carbon atoms.

9. The photoacid generator according to claim 1, wherein:
the monocyclic or polycyclic aromatic hydrocarbon group is an aryl group having a carbon number substituted with one or more electron withdrawing groups (EWG).

10. The photoacid generator according to claim 1, wherein:
the R is either a cyclic aliphatic hydrocarbon group or an aromatic hydrocarbon group.

11. The photoacid generator according to claim 1, wherein:
the R is a monocyclic aromatic hydrocarbon having 6 to 30 carbon atoms.

**12.** The photoacid generator according to claim 1, wherein:
the EWG is one or more electron withdrawing groups selected from the group consisting of a halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyl group; an aryloxy group; an alkylthioxy group; an arylthioxy group; an alkyl sulphoxy group; an aryl sulfoxy group; a silyl group; a boron group; and a cycloalkyl group.

**13.** The photoacid generator according to claim 1, wherein:
the EWG is one or more electron withdrawing groups selected from the group consisting of a nitro group, a halogen, a fluoroalkyl group, a cyano group, and a sulfonyl group.

**14.** The photoacid generator according to claim 1, wherein:

the R is a monocyclic aromatic hydrocarbon, and
the EWG is one or more selected from a halogen group, a nitro group, a halogen, a fluoroalkyl group, a cyano group, and a sulfonyl group.

**15.** The photoacid generator according to claim 1, wherein:

the photoacid generator is a compound represented by the following Chemical Formula 3:

[Chemical Formula 3]

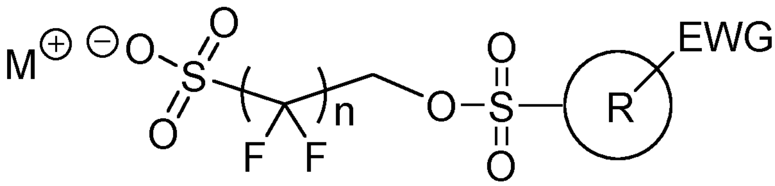

wherein, in Chemical Formula 3,
n is an integer of 1 to 10, and
R, EWG and $M^+$ are as defined above.

**16.** The photoacid generator according to claim 1, wherein:
the anion represented by Chemical Formula 1 is any one selected from the group consisting of:

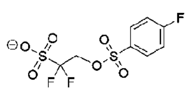 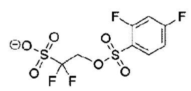 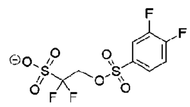 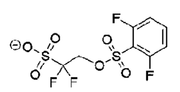 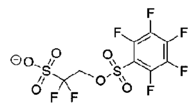

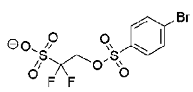 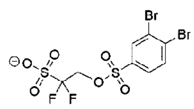 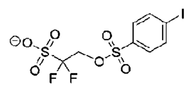 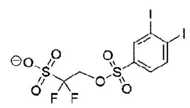 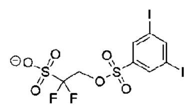

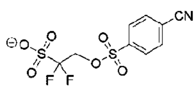 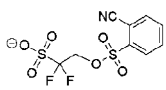 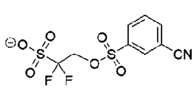 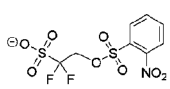 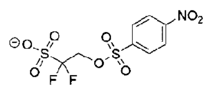

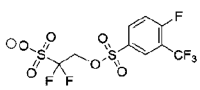 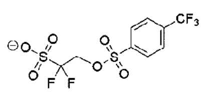 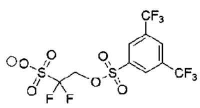 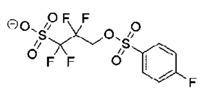 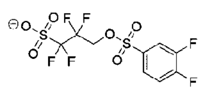

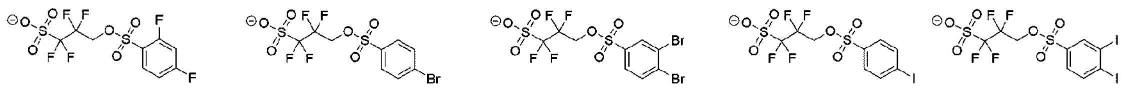
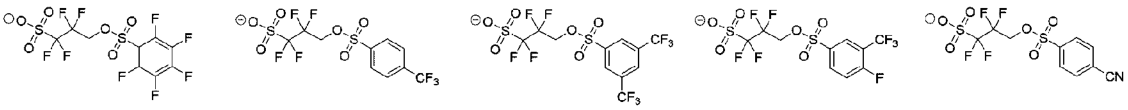
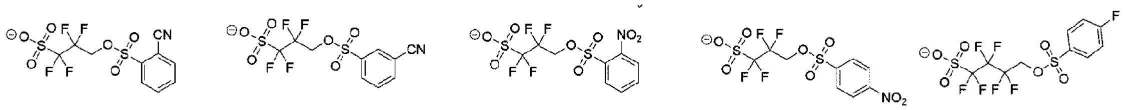
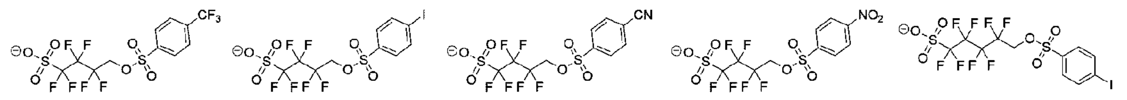
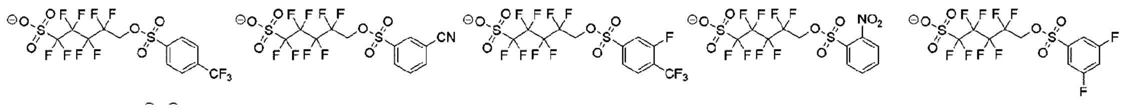
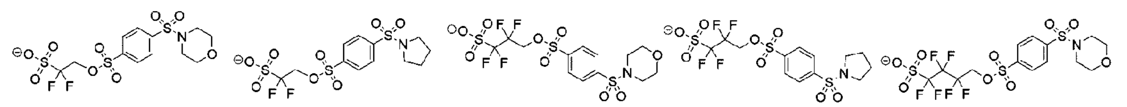
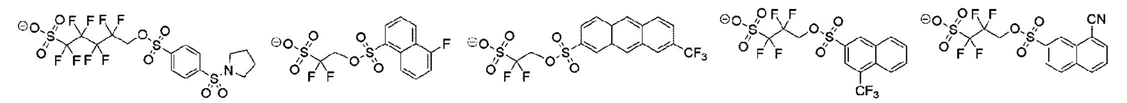
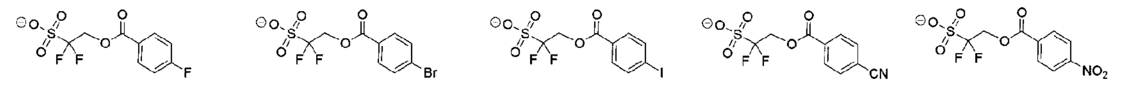
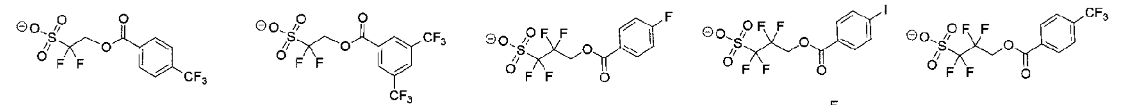
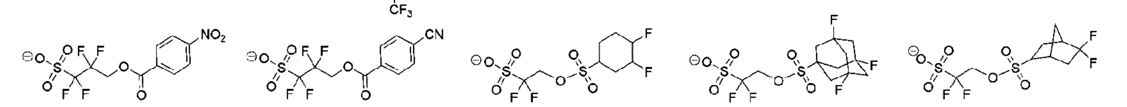

17. The photoacid generator according to claim 1, wherein:
the cation represented by Chemical Formula 2 is any one selected from the group consisting of:

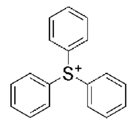
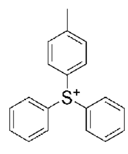
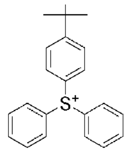
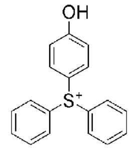
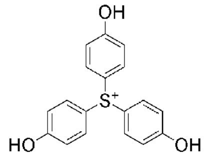
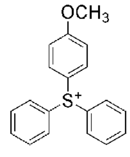

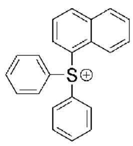
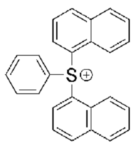
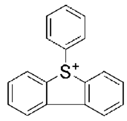
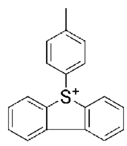
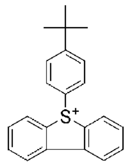
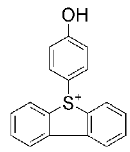

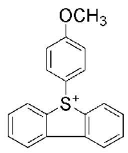
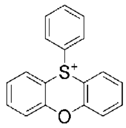
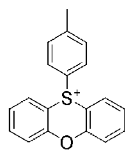
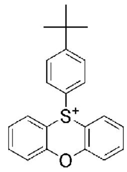
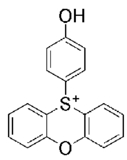
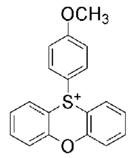

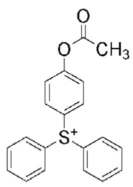
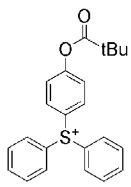
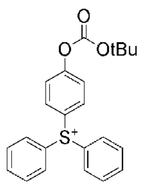
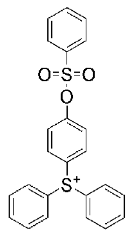
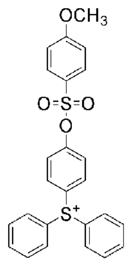
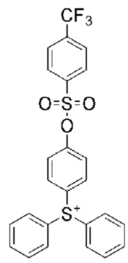

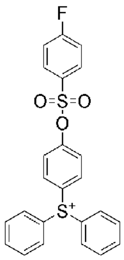
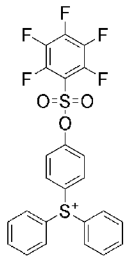
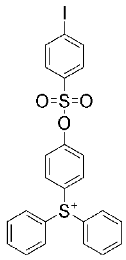
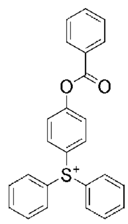
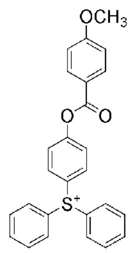
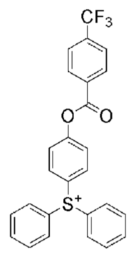

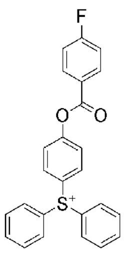
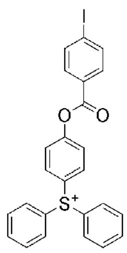
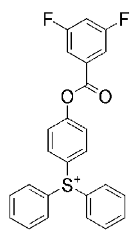
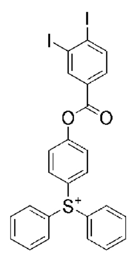
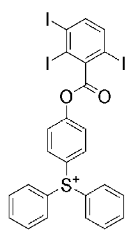
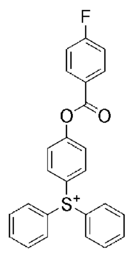

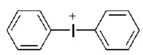
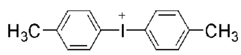
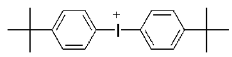
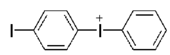

**18.** A photoresist composition comprising:

the photoacid generator according to claim 1;
a base polymer;
an acid diffusion inhibitor; and
an organic solvent.

【FIG. 1】

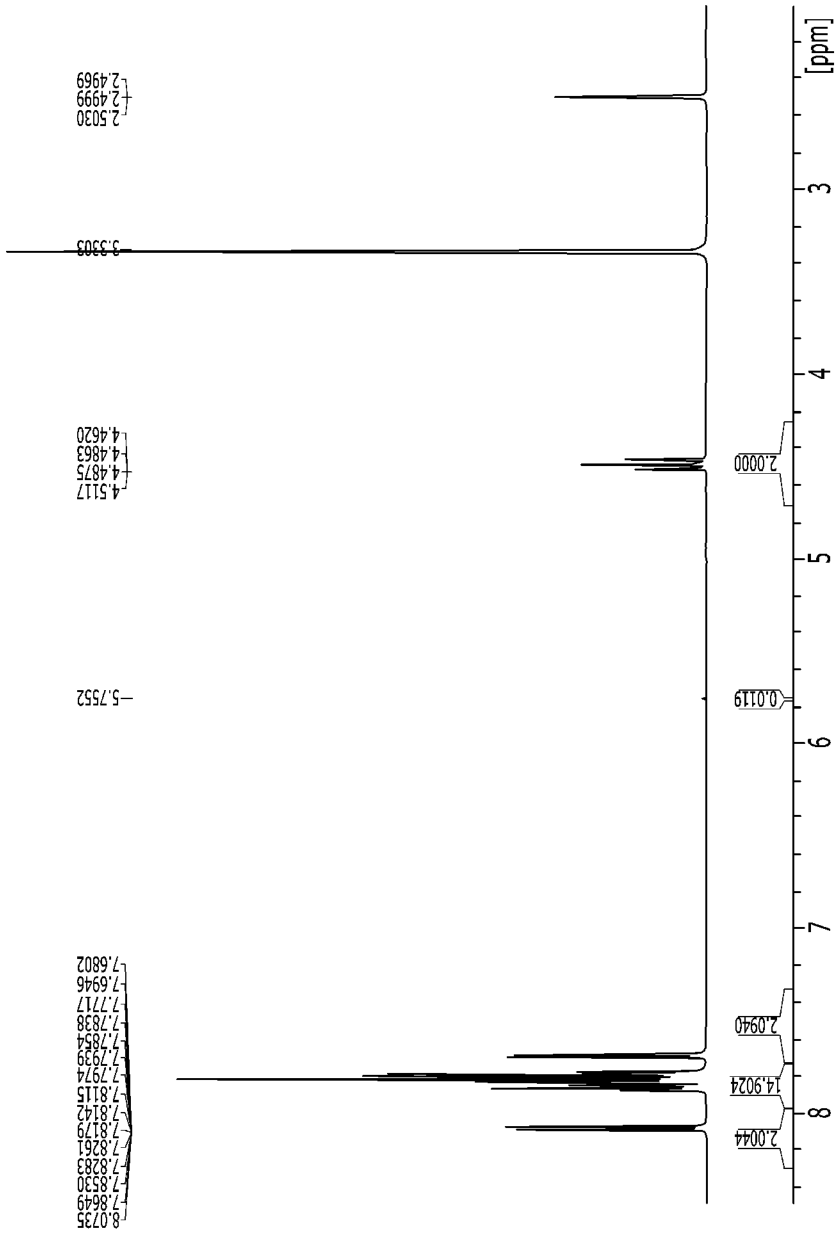

2.4969
2.4999
2.5030
3.3303
4.4620
4.4863
4.4875
4.5117
5.7552
7.6802
7.6946
7.7717
7.7838
7.7854
7.7939
7.7974
7.8115
7.8142
7.8179
7.8261
7.8283
7.8530
7.8649
8.0735
2.0000
0.0119
2.0940
14.9024
2.0044
3
4
5
6
7
8
[ppm]

【FIG. 2】

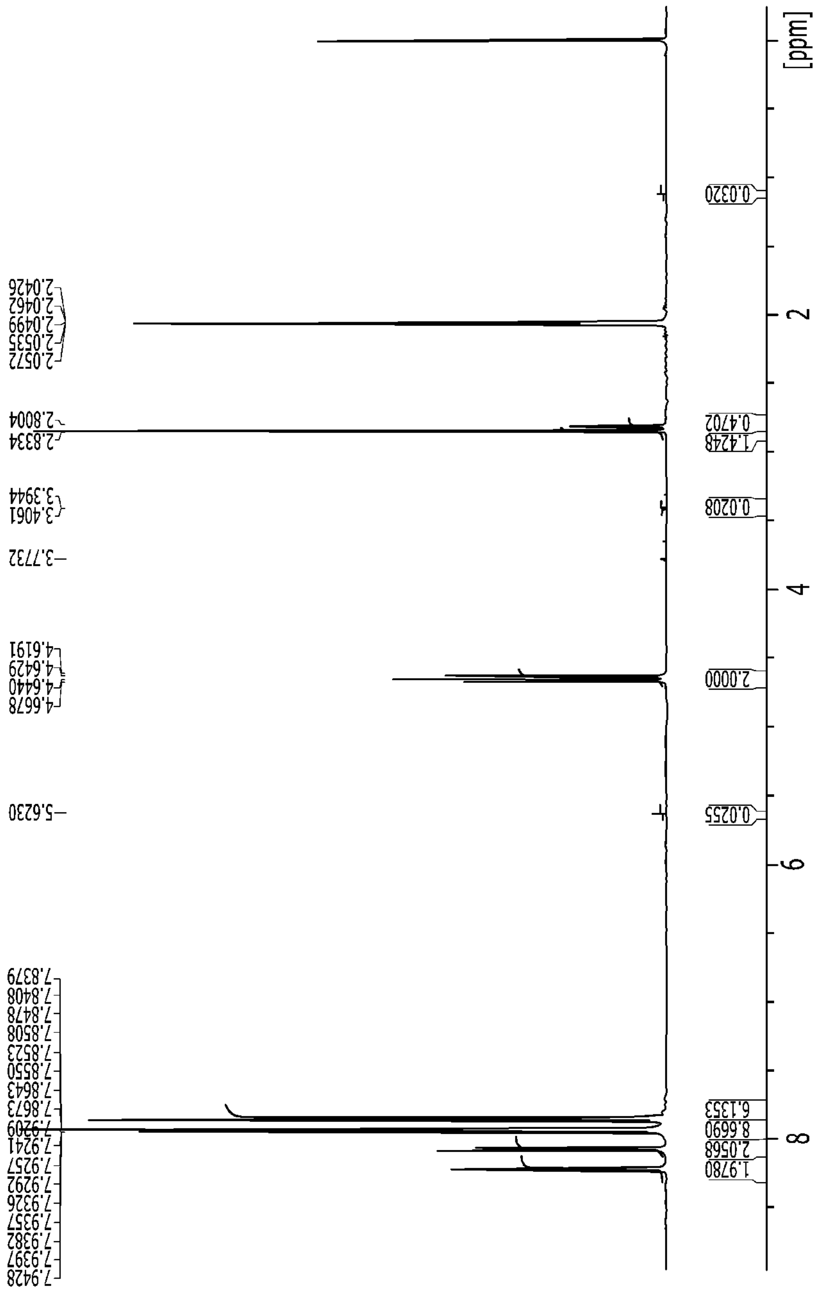
2.0426
2.0462
2.0499
2.0535
2.0572
2.8004
2.8334
3.3944
3.4061
3.7732
4.6191
4.6429
4.6440
4.6678
5.6230
7.8379
7.8408
7.8478
7.8508
7.8523
7.8550
7.8643
7.8673
7.9209
7.9241
7.9257
7.9292
7.9326
7.9357
7.9382
7.9397
7.9428
0.0320
0.4702
1.4248
0.0208
2.0000
0.0255
6.1353
8.6690
2.0568
1.9780
[ppm]
2
4
6
8

【FIG. 3】

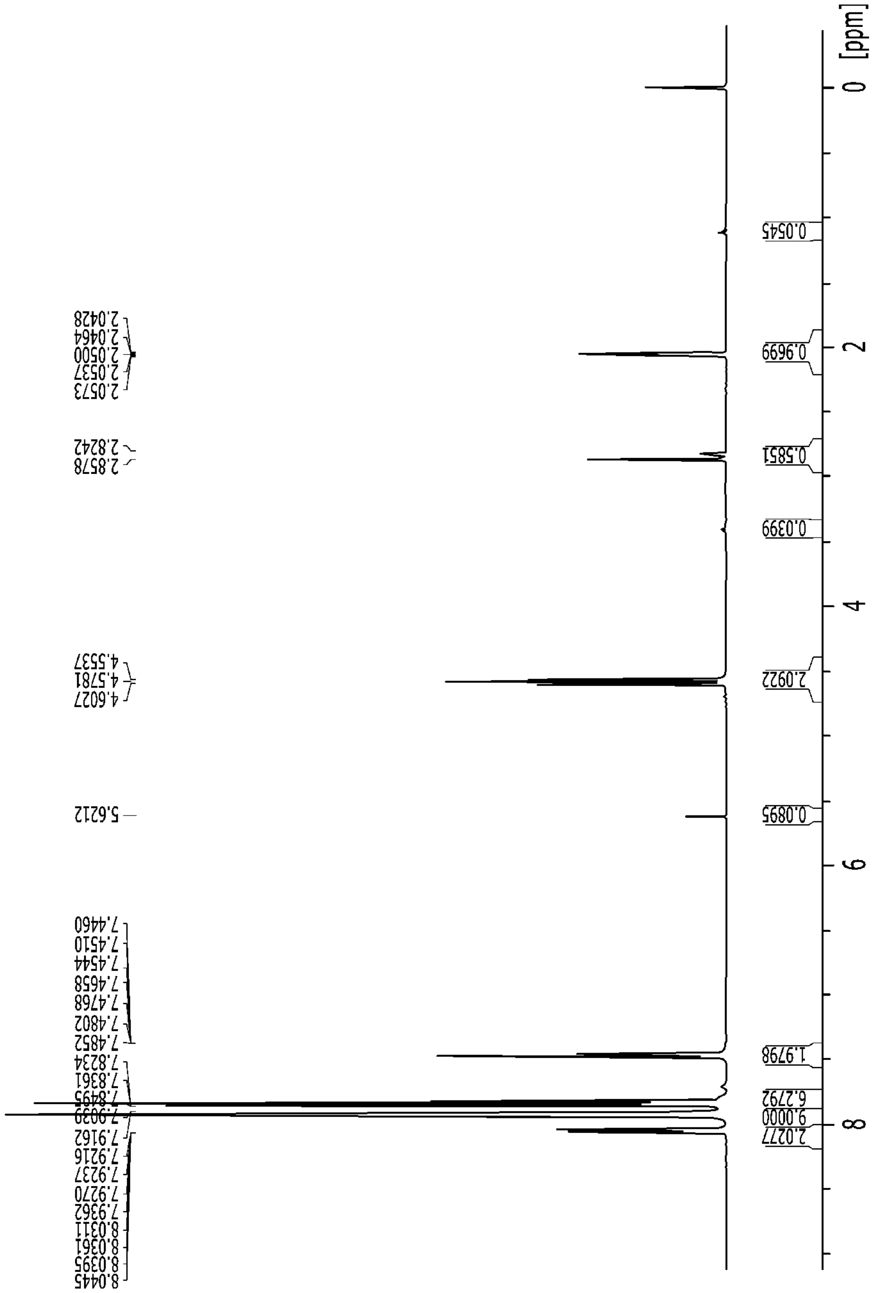
2.0428
2.0464
2.0500
2.0537
2.0573
2.8242
2.8578
4.5537
4.5781
4.6027
5.6212
7.4460
7.4510
7.4544
7.4658
7.4768
7.4802
7.4852
7.8234
7.8361
7.8495
7.9030
7.9162
7.9216
7.9237
7.9270
7.9362
8.0311
8.0361
8.0395
8.0445
0.0545
0.9699
0.5851
0.0399
2.0922
0.0895
1.9798
6.2792
9.0000
2.0277
0
2
4
6
8
[ppm]

**INTERNATIONAL SEARCH REPORT**

International application No. **PCT/KR2021/019566**

**A. CLASSIFICATION OF SUBJECT MATTER**

**G03F 7/004**(2006.01)i; **C07C 323/20**(2006.01)i; **C07C 323/21**(2006.01)i

According to International Patent Classification (IPC) or to both national classification and IPC

**B. FIELDS SEARCHED**

Minimum documentation searched (classification system followed by classification symbols)

G03F 7/004(2006.01); C07C 309/63(2006.01); C07C 309/71(2006.01); C08F 220/10(2006.01); C08F 232/00(2006.01); G03F 7/032(2006.01); G03F 7/033(2006.01); G03F 7/038(2006.01); H01L 21/027(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 광산발생제(acid generator), 음이온(anion), 양이온(cation), 술포네이트(sulfonate), 카보네이트(carbonate), 술포닐옥시기(sulfonyloxy), 카보닐옥시(carbonyloxy), 산확산 억제제(acid diffusion inhibitor)

**C. DOCUMENTS CONSIDERED TO BE RELEVANT**

| Category* | Citation of document, with indication, where appropriate, of the relevant passages | Relevant to claim No. |
|---|---|---|
| X<br>A | KR 10-2020-0112735 A (SHIN-ETSU CHEMICAL CO., LTD.) 05 October 2020 (2020-10-05)<br>See claim 1; and paragraphs [0133], [0134], [0152], [0153] and [0179]. | 1-6,9-14,16-18<br>7,8,15 |
| X | KR 10-2011-0013869 A (KOREA KUMHO PETROCHEMICAL CO., LTD.) 10 February 2011 (2011-02-10)<br>See claims 5-10; and paragraphs [0085]-[0136] and [0196]-[0200]. | 1-8,12,13,15,17,18 |
| X | KR 10-2010-0048047 A (KOREA KUMHO PETROCHEMICAL CO., LTD.) 11 May 2010 (2010-05-11)<br>See claims 1-16; and paragraphs [0117] and [0206]-[0214]. | 1-6,9-14,16-18 |
| X | KR 10-2018-0136396 A (SHIN-ETSU CHEMICAL CO., LTD.) 24 December 2018 (2018-12-24)<br>See claims 1-15. | 1-6,9-14,16-18 |

✓ Further documents are listed in the continuation of Box C. ✓ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **02 September 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/019566**

**C. DOCUMENTS CONSIDERED TO BE RELEVANT**

| Category* | Citation of document, with indication, where appropriate, of the relevant passages | Relevant to claim No. |
|---|---|---|
| A | KR 10-2011-0035976 A (FUJIFILM CORPORATION) 06 April 2011 (2011-04-06)<br>See paragraphs [0553]-[0556]. | 1-18 |

Form PCT/ISA/210 (second sheet) (July 2019)

**INTERNATIONAL SEARCH REPORT**
**Information on patent family members**

International application No.
**PCT/KR2021/019566**

| Patent document cited in search report | Publication date (day/month/year) | Patent family member(s) | Publication date (day/month/year) |
|---|---|---|---|
| KR 10-2020-0112735 A | 05 October 2020 | JP 2020-154210 A | 24 September 2020 |
| | | JP 7096188 B2 | 05 July 2022 |
| | | KR 10-2411008 B1 | 17 June 2022 |
| | | TW 202040273 A | 01 November 2020 |
| | | TW I714477 B | 21 December 2020 |
| | | US 2020-0301275 A1 | 24 September 2020 |
| KR 10-2011-0013869 A | 10 February 2011 | CN 101987880 A | 23 March 2011 |
| | | CN 101987880 B | 13 August 2014 |
| | | KR 10-1111491 B1 | 14 March 2012 |
| | | SG 168462 A1 | 28 February 2011 |
| | | SG 190581 A1 | 28 June 2013 |
| | | TW 201107353 A | 01 March 2011 |
| | | TW I481627 B | 21 April 2015 |
| KR 10-2010-0048047 A | 11 May 2010 | CN 101727004 A | 09 June 2010 |
| | | CN 101727004 B | 22 May 2013 |
| | | JP 2010-106236 A | 13 May 2010 |
| | | JP 5149236 B2 | 20 February 2013 |
| | | KR 10-0998503 B1 | 07 December 2010 |
| | | SG 161145 A1 | 27 May 2010 |
| | | TW 201016649 A | 01 May 2010 |
| | | TW I421236 B | 01 January 2014 |
| | | US 2010-0113818 A1 | 06 May 2010 |
| | | US 8026390 B2 | 27 September 2011 |
| KR 10-2018-0136396 A | 24 December 2018 | JP 2019-003175 A | 10 January 2019 |
| | | JP 6973279 B2 | 24 November 2021 |
| | | KR 10-2148074 B1 | 25 August 2020 |
| | | TW 201904939 A | 01 February 2019 |
| | | TW I672288 B | 21 September 2019 |
| | | US 10725378 B2 | 28 July 2020 |
| | | US 2018-0364574 A1 | 20 December 2018 |
| KR 10-2011-0035976 A | 06 April 2011 | JP 2011-095700 A | 12 May 2011 |
| | | JP 5675125 B2 | 25 February 2015 |
| | | KR 10-1774311 B1 | 04 September 2017 |
| | | TW 201127796 A | 16 August 2011 |
| | | TW I486327 B | 01 June 2015 |
| | | US 2011-0076615 A1 | 31 March 2011 |
| | | US 8940471 B2 | 27 January 2015 |

Form PCT/ISA/210 (patent family annex) (July 2019)